# EUROPEAN PATENT APPLICATION

(11) **EP 2 117 009 A1**
(43) Date of publication of application: **11.11.2009**
(21) Application number: 08721142.1
(22) Date of filing: 26.02.2008
(51) Int. Cl.: H01B 3/30, C09D 5/25, C09D 149/00, C09D 157/00, H01L 21/312

(54) **ORGANIC INSULATING MATERIAL, VARNISH FOR ORGANIC INSULATING FILM USING THE SAME, ORGANIC INSULATING FILM AND SEMICONDUCTOR DEVICE**

(30) Priority: 28.02.2007 JP 2007048890; 30.11.2007 JP 2007309715
(71) Applicant: Sumitomo Bakelite Co., Ltd., Shinagawa-ku Tokyo 140-0002 (JP)
(72) Inventor: MATSUTANI, Mihoko, Tokyo 140-0002 (JP); IZUMI, Atsushi, Tokyo 140-0002 (JP); SANO, Yohko, Tokyo 140-0002 (JP); FUJITA, Kazuyoshi, Tokyo 140-0002 (JP)
(74) Representative: Cohausz & Florack
(86) International application number: PCT/JP2008/053722
(87) International publication number: WO 2008/105551

(57) **Abstract**

There are provided organic insulating materials exhibiting low permittivity, high heat resistance and high mechanical strength, as well as organic insulating films with low permittivity, high heat resistance and high mechanical strength that employ the same, and semiconductor devices comprising the foregoing. An organic insulating material comprising a compound represented by general formula (1), or a polymer obtained by polymerizing a compound represented by general formula (1), or a mixture of a compound represented by general formula (1) and the polymer. 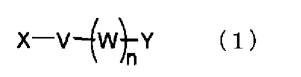 (In formula (1), X and Y each independently represent one or more groups with polymerizable functional groups. V and W each represent a group having an adamantane or polyamantane structure, and they may be the same or different. The letter n represents an integer of 0 or greater.)

## Description

### Technical Field

The present invention relates to organic insulating materials, varnishes for organic insulating film employing them, organic insulating films and semiconductor devices.

### Background Art

With increasing high integration, high speed and high performance of semiconductor devices in the field of electronic materials in recent years, the delay times caused by greater interconnect resistance and greater electric capacity of semiconductor integrated circuits are becoming more serious problems. In order to mitigate the delay times and increase semiconductor device speeds, it has become necessary to use low permittivity insulating films in circuits. Heating steps are carried out in the manufacture of semiconductor devices, and high heat resistance is therefore required for the insulating films. It has therefore been desirable to develop materials exhibiting both low permittivity and high heat resistance.

Polyimide resins and the like are known as organic insulating materials and are disclosed, for example, in Japanese Unexamined Patent Publication HEI No. 5-121396. However, since resin films composed of polyimide resins generally have relatively low heat resistance, high permittivity and also high hygroscopicity, they have only been used in certain types of semiconductor elements such as bipolar semiconductor elements, for reasons of reliability.

As organic insulating materials there are known compositions obtained by polymerizing 1,2-diiodobenzene and 4,4-diethynyldiphenyl ether in the presence of a palladium catalyst, as disclosed in Japanese Unexamined Patent Publication No. 2002-322246, for example. Resin films composed of such compositions, however, have not exhibited satisfactory values for their mechanical strength and electrical characteristics.

### Disclosure of the Invention

In light of these circumstances, it is an object of the present invention to provide organic insulating materials exhibiting low permittivity, high heat resistance and high mechanical strength, as well as to provide organic insulating films with low permittivity, high heat resistance and high mechanical strength that employ the same, and semiconductor devices comprising the foregoing.

Specifically, the invention comprises the following aspects (1) to (10).
(1) An organic insulating material comprising a compound represented by general formula (1), or a polymer obtained by polymerizing a compound represented by general formula (1), or a mixture of a compound represented by general formula (1) and said polymer. 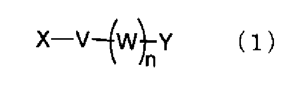 (In formula (1), X and Y each independently represent one or more groups with polymerizable functional groups. V and W each represent a group having an adamantane or polyamantane structure, and they may be the same or different. The symbol n represents an integer of 0 or greater.)
(2) An organic insulating material according to (1), wherein the compound represented by general formula (1) is a compound represented by general formula (2). 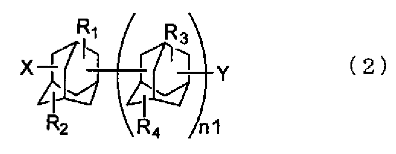 (In formula (2), X and Y each independently represent one or more groups with polymerizable functional groups. R₁-R₄ each independently represent hydrogen or an organic group, and they may be the same or different from each other. The symbol n1 represents an integer of 0 or greater. When n1 is an integer of 2 or greater, R₃ and R₄ may be the same or different in each adamantane structure.)
(3) An organic insulating material according to (2), wherein the compound represented by general formula (2) is a biadamantane compound.
(4) An organic insulating material according to (3), wherein the biadamantane compound has a 1,1'-biadamantane skeleton.
(5) An organic insulating material according to any one of (1) to (4), wherein the compound represented by general formula (1) has a group with a polymerizable unsaturated bond group as X and/or Y.
(6) An organic insulating material according to (5), wherein the polymerizable unsaturated bond group is an acetylene bond group or vinyl bond group.
(7) An organic insulating material according to (6), wherein X and Y in the compound represented by general formula (1) each independently have one or more groups selected from among groups represented by general formulas (3)-(8).

   -Z(̵O-R₅-C≡C-R₆)ₘ₁ (3)

   (In formula (3), Z represents a single bond or an aromatic group, R₅ represents an aliphatic group, and R₆ represents hydrogen or an organic group. When Z is a single bond m1 is 1, and when Z is an aromatic group m1 is 1 or 2.)

   -C=C-R₆ (4)

   (In formula (4), R₆ represents hydrogen or an organic group.) 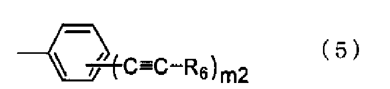 (In formula (5), R₆ represents hydrogen or an organic group. The symbol m2 represents an integer of 1-5.) 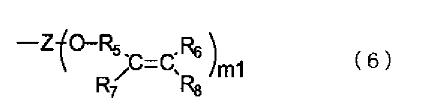 (In formula (6), Z represents a single bond or an aromatic group, R₅ represents an aliphatic group and R₆-R₈ represent hydrogen or organic groups and each independently may be the same or different. When Z is a single bond m1 is 1, and when Z is an aromatic group m1 is 1 or 2.) 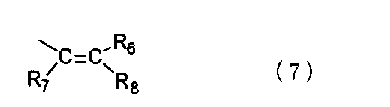 (In formula (7), R₆-R₈ represent hydrogen or organic groups and each independently may be the same or different.) 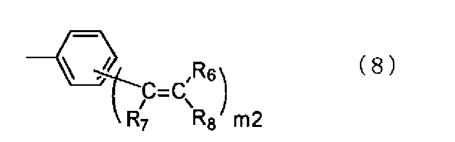 (In formula (8), R₆-R₈ represent hydrogen or organic groups and each independently may be the same or different. The symbol m2 represents an integer of 1-5.)
(8) A varnish for organic insulating film comprising an organic insulating material according to any one of (1) to (7) and an organic solvent.
(9) An organic insulating film obtained by using an organic insulating material according to any one of (1) to (7) or a varnish for organic insulating film according to (8), for crosslinking reaction by heating, active energy beam irradiation, or heating and active energy beam irradiation.
(10) A semiconductor device provided with an organic insulating film according to (9).

### Brief Description of the Drawings

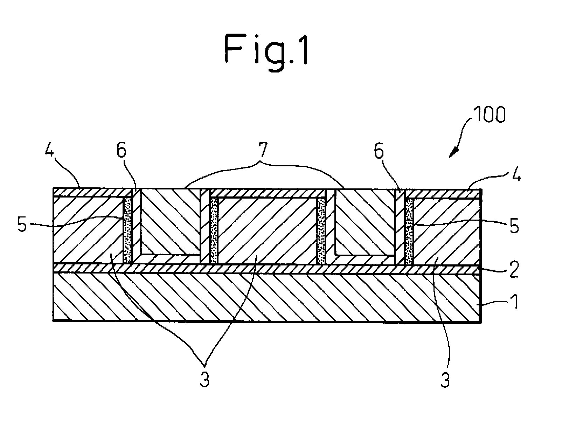

Fig. 1 is a schematic cross-sectional view showing an example of a semiconductor device according to the invention.

### Best Mode for Carrying Out the Invention

An organic insulating material according to the invention comprises a compound represented by general formula (1) above, or a polymer obtained by polymerizing a compound represented by general formula (1), or a mixture of a compound represented by general formula (1) and said polymer, whereby it is possible to obtain organic insulating films with excellent heat-resistant properties, mechanical properties and electrical characteristics.

A varnish for organic insulating film according to the invention comprises the aforementioned organic insulating material.

An organic insulating film according to the invention is obtained using the aforementioned organic insulating material or varnish for organic insulating film, by crosslinking reaction using the compound represented by general formula (1) above, the polymer obtained by polymerizing a compound represented by general formula (1), or the mixture of a compound represented by general formula (1) and said polymer, in the organic insulating material, whereby it is possible to obtain an organic insulating film with excellent heat-resistant properties, mechanical properties and electrical characteristics.

A semiconductor device according to the invention comprises the aforementioned organic insulating film. It is thus possible to obtain a highly reliable semiconductor device with reduced wiring delay.

The organic insulating material of the invention will be explained first.

The compound represented by general formula (1) used for the invention is characterized by comprising an adamantane or polyamantane structure with at least two polymerizable functional groups.

As groups with adamantane or polyamantane structures there may be mentioned adamantane structures and polyamantane structures themselves, as well as polyadamantane structures having a skeleton with a plurality of the aforementioned adamantane structures linked together, and poly(polyamantane) structures having a skeleton with a plurality of the aforementioned polyamantane structures linked together.

The compound represented by general formula (1) has a structure represented by the following formula. 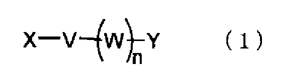 (In formula (1), X and Y each independently represent one or more groups with polymerizable functional groups. V and W each represent a group having an adamantane or polyamantane structure, and they may be the same or different. The symbol n represents an integer of 0 or greater.)

The compound having a structure represented by formula (1) above comprises a group with an adamantane or polyamantane structure as V and W, but it may have both a group with an adamantane structure and with a polyamantane structure. Preferred for V and W in formula (1) are polyadamantane compounds represented by the following formula (2) and poly(polyamantane) compounds represented by formula (9), for the adamantane or polyamantane structure, particularly from the viewpoint of obtaining low permittivity. 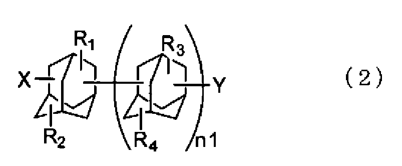 (In formula (2), X and Y each independently represent a group with a polymerizable functional group. R₁-R₄ each represent hydrogen or an organic group. The symbol n1 is the same as n in formula (1) above.) 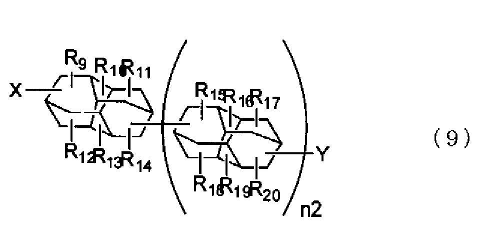 (In formula (9), X and Y each independently represent a group with a polymerizable functional group. R₉-R₂₀ each represent hydrogen or an organic group. The symbol n2 is the same as n in general formula (1) above.)

In the compound represented by general formula (1), W has n number of adamantane structures or polyamantane structures where the number n is 0 or greater, and although there is no particular upper limit, the number of adamantane or polyamantane structures in the compound represented by general formula (1) is preferably no greater than 10, i.e., n is preferably no greater than 9, from the viewpoint of solubility in organic solvents when it is to be used as a varnish. When the compound represented by general formula (1) is used as a copolymer, the number is preferably no greater than 4, i.e., the value of n is preferably no greater than 3, from the viewpoint of solubility in solvents.

The polyamantane structure referred to here may be a diamantane structure, triamantane structure, tetraamantane structure, pentaamantane structure, hexaamantane structure or the like.

As specific examples of adamantane skeletons comprising a plurality of adamantane structures linked together there may be mentioned biadamantane skeletons such as 1,1'-biadamantane skeleton, 2,2'-biadamantane skeleton and 1,2'-biadamantane skeleton, triadamantane skeletons such as 1,1':3',1''-triadamantane skeleton, 1,2':5',1''-triadamantane skeleton, 1,2':4',1''-triadamantane skeleton and 2,2':4',2''-triadamantane skeleton, tetraadamantane skeletons such as 1,1' :3',1'':3'',1'''-tetraadamantane skeleton, 1,2':5',1'':3'',1'''-tetraadamantane skeleton, 1,2':4',1'':3'',1'''-tetraadamantane skeleton, 1,1':4',1'':4'',1'''-tetraadamantane skeleton and 1,1':3',1'':3'',2'''-tetraadamantane skeleton, and pentaadamantane skeletons such as 1,1': 3',1'':3'',1''': 3''',1''''-pentaadamantane skeleton, 1,1' :4',1'':3'',1''':3''',1''''-pentaadamantane skeleton, 1,1' :4',1'':4'',1''':3''',1''''-pentaadamantane skeleton and 1,1' :3',1'':4'',2''':5''',1''''-pentaadamantane skeleton. Of these, biadamantane compounds with biadamantane skeletons are preferred from the standpoint of solubility in solvents. As biadamantane skeletons there may be mentioned 1,1'-biadamantane skeleton, 2,2'-biadamantane skeleton and 1,2'-biadamantane skeleton, with 1,1'-biadamantane skeleton being preferred to obtain an organic insulating film with more excellent heat resistance.

As specific examples of poly(polyamantane) skeletons comprising a plurality of polyamantane structures linked together, there may be mentioned skeletons comprising a plurality of diamantane structures linked together such as bi(diamantane) skeleton, tri(diamantane) skeleton, tetra(diamantane) skeleton and penta(diamantane) skeleton, skeletons comprising a plurality of triamantane structures linked together such as bi(triamantane) skeleton, tri(triamantane) skeleton, tetra(triamantane) skeleton and penta(triamantane) skeleton, and skeletons comprising a plurality of tetraamantane structures linked together such as bi(tetraamantane) skeleton, tri(tetraamantane) skeleton, tetra(tetraamantane) skeleton and penta(tetraamantane) skeleton.

Specific examples of polyamantane structures include those with diamantane skeletons, among which there may be mentioned bi(diamantane) skeletons such as 4,4'-bi(diamantane) skeleton, 3,3'-bi(diamantane) skeleton and 3,4'-bi(diamantane) skeleton, tri(diamantane) skeletons such as 4,4':9',4''-tri(diamantane) skeleton, 4,3':9',4''-tri(diamantane) skeleton, 4,3':8',4''-tri(diamantane) skeleton and 3,3':8',3''-tri(diamantane) skeleton, and tetra(diamantane) skeletons such as 4,4':9',4'':9'',4'''-tetra(diamantane) skeleton, 4,3':9',4'':9'',4'''-tetra(diamantane) skeleton, 4,4':8',4'':8'',4'''-tetra(diamantane) skeleton and 4,4':9',4'':9'',3'''-tetra(diamantane) skeleton. Of these, compounds with bi(diamantane) skeletons are preferred from the standpoint of solubility in solvents. More preferred are 4,4'-bi(diamantane) skeletons in order to obtain insulating films with more excellent heat resistance.

X and Y in general formula (1) represent one or more groups comprising polymerizable functional groups, and as polymerizable functional groups there may be mentioned radical-polymerizing functional groups, ion-polymerizable functional groups, coordination-polymerizable functional groups, photopolymerizable functional groups, radiation-polymerizable functional groups, plasma-polymerizable functional groups, and group transfer polymerizable functional groups. As specific examples there may be mentioned acetylene bond groups, vinyl bond groups, and cyano, carbonyl, amino group, carboxyl and nitro groups. The compound represented by general formula (1) preferably has a group comprising an unsaturated bond group as the polymerizable functional group in X or Y, or in both X and Y, to obtain an organic insulating material with more excellent heat resistance. As such unsaturated bond groups there may be mentioned acetylene bond groups, vinyl bond groups and cyano groups, with acetylene bond groups and vinyl bond groups being preferred.

As groups with acetylene bond groups there may be mentioned organic groups including substituted and unsubstituted ethynyl, and aliphatic groups and aromatic groups with substituted or unsubstituted ethynyl, or organic groups including aliphatic groups and aromatic groups with substituted or unsubstituted alkynyloxy groups, and as groups with vinyl bonds there may be mentioned organic groups including substituted and unsubstituted alkenyl or aliphatic groups and aromatic groups with substituted or unsubstituted alkenyl groups. As aliphatic groups in the groups with an acetylene bond group and groups with a vinyl bond group, there may be mentioned straight-chain aliphatic groups such as methyl and ethyl and cyclic aliphatic groups such as cyclohexyl and adamantyl, while as aromatic groups there may be mentioned phenyl, naphthyl and fluorenyl, although there is no limitation to these groups.

Also, as substituents in the groups with an acetylene bond group and groups with a vinyl bond group, there may be mentioned, but not limited to, organic groups such as straight-chain aliphatic groups, cyclic aliphatic groups and aromatic groups, where straight-chain aliphatic groups include methyl and ethyl, cyclic aliphatic groups include cyclohexyl and adamantyl, and aromatic groups include phenyl, naphthyl and fluorenyl.

As specific examples of groups with such unsaturated bond groups there are preferred groups represented by general formulas (3)-(8). Groups represented by general formulas (4), (5), (7) and (8) are more preferred.

The compounds represented by general formula (1) may have substituents that are the same or different for each adamantane structure bridgehead position or polyamantane structure bridgehead position. Specifically, R₁-R₄ in general formula (2) are each independently hydrogen or an organic group, and they may be the same or different. When n1 is an integer of 2 or greater, R₃ and R₄ may be the same or different in each adamantane structure. In the poly(polyamantane) structure compounds of the compounds represented by general formula (1), using compounds represented by general formula (9) as an example, R₉-R₂₀ are each independently hydrogen or an organic group, and may be the same or different. When n2 is an integer of 2 or greater, R₉-R₂₀ may be the same or different in each polyamantane structure.

As organic groups for R₁-R₄ and R₉-R₂₀ there may be mentioned aliphatic groups and aromatic groups, where the aliphatic groups may be straight-chain aliphatic groups, cyclic aliphatic groups or the like, specific examples of straight-chain aliphatic groups including methyl, ethyl, propyl, butyl group and hexyl, and specific examples of cyclic aliphatic groups including cyclohexyl, bicyclo[2,2,1]heptyl group and adamantyl. As aromatic groups there may be mentioned phenyl, naphthyl, anthracenyl, phenanthrenyl, polycyclic aromatic groups with 4 or more aromatic rings, fluorenyl, diphenylfluorenyl, biphenyl and the like, with no limitation to these. If the straight-chain aliphatic groups are methyl or ethyl, for example, the solubility in organic solvents and heat resistance can be improved. The hydrogens in these organic groups may also be replaced by fluorine atoms, methyl, methoxy or trifluoromethyl groups. R₁-R₄ and R₉-R₂₀ may also be groups containing polymerizable functional groups.

The groups represented by general formula (3) above will be explained first.

The groups represented by general formula (3) comprise a single bond or aromatic group as Z, and as aromatic groups there may be mentioned phenylene, naphthylene, anthracenylene, phenanthrenylene, polycyclic aromatic groups with 4-6 aromatic rings, fluorenylene, diphenylfluorenylene, biphenylene and the like, with no limitation to these. The hydrogens in these aromatic groups may also be replaced by fluorine atoms, methyl, methoxy or trifluoromethyl groups.

When Z in the group represented by general formula (3) is a single bond m1 will be 1, and when Z is an aromatic group m1 will be 1 or 2.

R₅ in the group represented by general formula (3) comprises an aliphatic group, and as aliphatic groups there may be mentioned C1-10 straight-chain aliphatic groups such as methylene, ethylene, propylene, butylene, hexylene, octylene and decylene, with no limitation to these. The hydrogens in these aliphatic groups may be replaced by halogen groups such as fluorine atoms and trifluoromethyl; C1-5 alkyl groups such as methyl, ethyl, propyl, isopropyl, butyl, isobutyl, t-butyl and pentyl; or C1-5 alkoxy groups such as methoxy, ethoxy, propoxy, isopropoxy, butoxy, isobutyloxy, t-butyloxy and pentyloxy.

Also, R₆ in the groups represented by general formula (3) comprises hydrogen or an organic group, and as organic groups there may be mentioned aliphatic groups such as straight-chain aliphatic groups and cyclic aliphatic groups, and aromatic groups. As straight-chain aliphatic groups there may be mentioned methyl, ethyl, propyl, butyl, hexyl, heptyl and octyl, and as cyclic aliphatic groups there may be mentioned cyclohexyl, cycloheptyl, bicyclo[2,2,1]heptyl and adamantyl. As aromatic groups there may be mentioned phenyl, naphthyl, anthracenyl, phenanthrenyl, phenoxyphenyl, polycyclic aromatic groups with 4 or more aromatic rings, fluorenyl, diphenylfluorenyl, biphenyl and the like, with no limitation to these. The hydrogens in these organic groups may also be replaced by fluorine atoms, methyl, methoxy, trifluoromethyl, adamantyl, phenyl or the like.

Concrete examples of groups represented by general formula (3) above will now be explained.

As specific examples of groups comprising acetylene bond groups represented by general formula (3), there may be mentioned alkynyloxy, alkynyloxyphenyl and bis(alkynyloxy)phenyl. As examples of alkynyloxy groups there may be mentioned 2-propynyloxy, 2-butynyloxy, 3-butynyloxy, 2-hexynyloxy, 3-hexynyloxy, 4-hexynyloxy, 5-hexynyloxy, 2-heptynyloxy, 3-heptynyloxy, 4-heptynyloxy, 5-heptynyloxy, 6-heptynyloxy, 1,1-dimethyl-2-propynyloxy and 1,1-diphenyl-2-propynyloxy; as examples of alkynyloxyphenyl groups there may be mentioned 4-(2-propynyloxy)phenyl, 4-(2-butynyloxy)phenyl, 4-(3-butynyloxy)phenyl, 4-(2-hexynyloxy)phenyl, 4-(3-hexynyloxy)phenyl, 4-(4-hexynyloxy)phenyl, 4-(5-hexynyloxy)phenyl, 4-(2-heptynyloxy)phenyl, 4-(3-heptynyloxy)phenyl, 4-(4-heptynyloxy)phenyl, 4-(5-heptynyloxy)phenyl, 4-(6-heptynyloxy)phenyl, 4-(1,1-dimethyl-2-propynyloxy)phenyl and 4-(1,1-diphenyl-2-propynyloxy)phenyl; and as examples of bis(alkynyloxy)phenyl groups there may be mentioned 2,4-bis(2-propynyloxy)phenyl, 2,4-bis(2-butynyloxy)phenyl, 2,4-bis(3-butynyloxy)phenyl, 2,4-bis(2-hexynyloxy)phenyl, 2,4-bis(3-hexynyloxy)phenyl, 2,4-bis(4-hexynyloxy)phenyl, 2,4-bis(5-hexynyloxy)phenyl, 2,4-bis(2-heptynyloxy)phenyl, 2,4-bis(3-heptynyloxy)phenyl, 2,4-bis(4-heptynyloxy)phenyl, 2,4-bis(5-heptynyloxy)phenyl, 2,4-bis(6-heptynyloxy)phenyl, 2,4-bis(1,1-dimethyl-2-propynyloxy)phenyl, 2,4-bis(1,1-diphenyl-2-propynyloxy)phenyl, 2,3-bis(2-propynyloxy)phenyl, 2,5-bis(2-propynyloxy)phenyl, 2,6-bis(2-propynyloxy)phenyl, 3,4-bis(2-propynyloxy)phenyl and 3,5-bis(2-propynyloxy)phenyl, with no limitation to these. The hydrogens in the aforementioned acetylene bond-containing groups may be replaced by fluorine atoms, methyl, trifluoromethyl, phenyl or the like.

Among these there are preferred 2-propynyloxy, (2-propynyloxy)phenyl and bis(2-propynyloxy)phenyl, and more preferably 2-propynyloxy, 4-(2-propynyloxy)phenyl and 2,4-bis(2-propynyloxy)phenyl, in order to obtain organic insulating films with more excellent heat resistance.

Of the compounds represented by general formula (1) above, specific examples of compounds with acetylene bond-containing groups represented by general formula (3) include those with alkynyloxy groups, specifically 2-propynyloxy groups, such as 4,9-bis(2-propynyloxy)diamantane, 2,4,7,9-tetrakis(2-propynyloxy)diamantane, 4,4'-bis(2-propynyloxy)-9,9'-bi(diamantane), 3,3'-bis(2-propynyloxy)-1,1'-biadamantane, 3,3',5,5'-tetramethyl-7,7'-bis(2-propynyloxy)-1,1'-biadamantane, 3,5-bis(2-propynyloxy)-1,1'-biadamantane, 3,5,3'-tris(2-propynyloxy)-1,1'-biadamantane, 3,3',5,5'-tetrakis(2-propynyloxy)-1,1'-biadamantane, 3,3',5,7-tetrakis(2-propynyloxy)-1,1'-biadamantane, 3,3',5,5',7-pentakis(2-propynyloxy)-1,1'-biadamantane, 3,3',5,5',7,7'-hexakis(2-propynyloxy)-1,1'-biadamantane, 3,3',5,5'-tetramethyl-7,7'-bis(2-propynyloxy)-1,1'-biadamantane, 3,3',5,5'-tetraphenyl-7,7'-bis(2-propynyloxy)-1,1'-biadamantane, 3,3'-bis(2-propynyloxy)-1,2'-biadamantane and 3,3'-bis(2-propynyloxy)-2,2'-biadamantane; those with alkynyloxyphenyl groups, specifically 2-propynyloxyphenyl groups such as 4,9-bis(4-(2-propynyloxy)phenyl)diamantane, 2,4,7,9-tetrakis(4-(2-propynyloxy)phenyl)diamantane, 4,4'-bis(4-(2-propynyloxy)phenyl)-9,9'-bi(diamantane), 3,3'-bis(4-(2-propynyloxy)phenyl)-1,1'-biadamantane, 3,3',5,5'-tetramethyl-7,7'-bis(4-(2-propynyloxy)phenyl)-1,1'-biadamantane, 3,5-bis(4-(2-propynyloxy)phenyl)-1,1'-biadamantane, 3,5,3'-tris(4-(2-propynyloxy)phenyl)-1,1'-biadamantane, 3,3',5,5'-tetrakis(4-(2-propynyloxy)phenyl)-1,1'-biadamantane, 3,3',5,7-tetrakis(4-(2-propynyloxy)phenyl)-1,1'-biadamantane, 3,3',5,5',7-pentakis(4-(2-propynyloxy)phenyl)-1,1'-biadamantane, 3,3',5,5',7,7'-hexakis(4-(2-propynyloxy)phenyl)-1,1'-biadamantane, 3,3',5,5'-tetramethyl-7,7'-bis(4-(2-propynyloxy)phenyl)-1,1'-biadamantane, 3,3',5,5'-tetraphenyl-7,7'-bis(4-(2-propynyloxy)phenyl)-1,1'-biadamantane, 3,3'-bis(4-(2-propynyloxy)phenyl)-1,2'-biadamantane and 3,3'-bis(4-(2-propynyloxy)phenyl)-2,2'-biadamantane; those with bis(alkynyloxy)phenyl groups, specifically bis(2-propynyloxy)phenyl groups such as 4,9-bis(2,4-bis(2-propynyloxy)phenyl)diamantane, 2,4,7,9-tetrakis(2,4-bis(2-propynyloxy)phenyl)diamantane, 4,4'-bis(2,4-bis(2-propynyloxy)phenyl)-9,9'-bi(diamantane), 3,3'-bis(2,4-bis(2-propynyloxy)phenyl)-1,1'-biadamantane, 3,3',5,5'-tetramethyl-7,7'-bis(2,4-bis(2-propynyloxy)phenyl)-1,1'-biadamantane, 3,5-bis(2,4-bis(2-propynyloxy)phenyl)-1,1'-biadamantane, 3,5,3'-tris(2,4-bis(2-propynyloxy)phenyl)-1,1'-biadamantane, 3,3',5,5'-tetrakis(2,4-bis(2-propynyloxy)phenyl)-1,1'-biadamantane, 3,3',5,7-tetrakis(2,4-bis(2-propynyloxy)phenyl)-1,1'-biadamantane, 3,3',5,5',7-pentakis(2,4-bis(2-propynyloxy)phenyl)-1,1'-biadamantane, 3,3',5,5',7,7'-hexakis(2,4-bis(2-propynyloxy)phenyl)-1,1'-biadamantane, 3,3',5,5'-tetramethyl-7,7'-bis(2,4-bis(2-propynyloxy)phenyl)-1,1'-biadamantane, 3,3',5,5'-tetraphenyl-7,7'-bis(2,4-bis(2-propynyloxy)phenyl)-1,1'-biadamantane, 3,3'-bis(2,4-bis(2-propynyloxy)phenyl)-1,2'-biadamantane and 3,3'-bis(2,4-bis(2-propynyloxy)phenyl)-2,2'-biadamantane, and the like, with no limitation to these. Particularly preferred of those mentioned above are 3,3',5,5'-tetramethyl-7,7'-bis(2-propynyloxy)-1,1-biadamantane, 3,3',5,5'-tetrakis(2-propynyloxy)-1,1-biadamantane, 3,3',5,5'-tetramethyl-7,7'-bis(4-(2-propynyloxy)phenyl)-1,1-biadamantane, 3,3',5,5'-tetrakis(4-(2-propynyloxy)phenyl)-1,1-biadamantane and 3,3',5,5'-tetramethyl-7,7'-bis(2,4-bis(2-propynyloxy)phenyl)-1,1-biadamantane. Although diamantane compounds were mentioned as examples of polyamantane structures among the specific examples of compounds represented by general formula (1), there is no limitation to such compounds. Also, biadamantane compounds were mentioned as polyadamantane structures, but they may also be polyadamantane compounds or poly(polyamantane) compounds wherein n is 2 or greater in general formula (1).

The groups represented by general formula (4) above will now be explained.

The groups represented by general formula (4) comprise hydrogen or an organic group as R₆. As organic groups for R₆ there may be mentioned the same ones as the organic groups for R₆ in the groups represented by general formula (3).

Concrete examples of groups represented by general formula (4) above will now be explained.

As specific examples of acetylene bond-containing groups represented by general formula (4) there may be mentioned ethynyl, methylethynyl, ethylethynyl, propylethynyl, butylethynyl, pentylethynyl, hexylethynyl, heptylethynyl, octylethynyl, adamantylethynyl, cycloheptylethynyl, cyclohexylethynyl, phenylethynyl, phenoxyphenylethynyl, naphthylethynyl and fluorenylethynyl, among which ethynyl, methylethynyl and phenylethynyl are preferred for superior heat resistance when used in an organic insulating film. The hydrogens in the aforementioned organic groups may be replaced by fluorine atoms, methyl, trifluoromethyl, phenyl or the like.

Specific examples of compounds with acetylene bond-containing groups represented by general formula (4), among the compounds represented by general formula (1) above, include those with hydrogen as R₆ such as 4,9-diethynyldiamantane, 2,4,7,9-tetraethynyldiamantane, 4,4'-diethynyl-9,9'-bi(diamantane), 3,3'-diethynyl-1,1'-biadamantane, 3,3',5,5'-tetramethyl-7,7'-diethynyl-1,1'-biadamantane, 3,5-diethynyl-1,1'-biadamantane, 3,5,3'-triethynyl-1,1'-biadamantane, 3,3',5,5'-tetraethynyl-1,1'-biadamantane, 3,3',5,7-tetraethynyl-1,1'-biadamantane, 3,3',5,5',7-pentaethynyl-1,1'-biadamantane, 3,3',5,5',7,7'-hexaethynyl-1,1'-biadamantane, 3,3',5,5'-tetraphenyl-7,7'-diethynyl-1,1'-biadamantane, 3,3'-diethynyl-1,2'-biadamantane and 3,3'-diethynyl-2,2'-biadamantane; those with methyl groups among the aforementioned organic groups as R₆, such as 4,9-bis(methylethynyl)diamantane, 2,4,7,9-tetrakis(methylethynyl)diamantane, 4,4'-bis(methylethynyl)-9,9'-bi(diamantane), 3,3'-bis(methylethynyl)-1,1'-biadamantane, 3,3',5,5'-tetramethyl-7,7'-bis(methylethynyl)-1,1'-biadamantane, 3,5-bis(methylethynyl)-1,1'-biadamantane, 3,5,3'-tris(methylethynyl)-1,1'-biadamantane, 3,3',5,5'-tetrakis(methylethynyl)-1,1'-biadamantane, 3,3',5,7-tetrakis(methylethynyl)-1,1'-biadamantane, 3,3',5,5',7-pentakis(methylethynyl)-1,1'-biadamantane, 3,3',5,5',7,7'-hexakis(methylethynyl)-1,1'-biadamantane, 3,3',5,5'-tetraphenyl-7,7'-bis(methylethynyl)-1,1'-biadamantane, 3,3'-bis(methylethynyl)-1,2'-biadamantane and 3,3'-bis(methylethynyl)-2,2'-biadamantane; and those with phenyl among the aforementioned organic groups as R₆, such as 4,9-bis(phenylethynyl)diamantane, 2,4,7,9-tetrakis(phenylethynyl)diamantane, 4,4'-bis(phenylethynyl)-9,9'-bi(diamantane), 3,3'-bis(phenylethynyl)-1,1'-biadamantane, 3,3',5,5'-tetramethyl-7,7'-bis(phenylethynyl)-1,1'-biadamantane, 3,5-bis(phenylethynyl)-1,1'-biadamantane, 3,5,3'-tris(phenylethynyl)-1,1'-biadamantane, 3,3',5,5'-tetrakis(phenylethynyl)-1,1'-biadamantane, 3,3',5,7-tetrakis(phenylethynyl)-1,1'-biadamantane, 3,3',5,5',7-pentakis(phenylethynyl)-1,1'-biadamantane, 3,3',5,5',7,7'-hexakis(phenylethynyl)-1,1'-biadamantane, 3,3',5,5'-tetraphenyl-7,7'-bis(phenylethynyl)-1,1'-biadamantane, 3,3'-bis(phenylethynyl)-1,2'-biadamantane and 3,3'-bis(phenylethynyl)-2,2'-biadamantane, with no limitation to these. Preferred among these are 3,3'-diethynyl-5,5',7,7'-tetramethyl-1,1'-biadamantane, 3,3',5,5'-tetraethynyl-1,1'-biadamantane, 3,3',5,5'-tetramethyl-7,7'-bis(methylethynyl)-1,1'-biadamantane and 3,3',5,5'-tetramethyl-7,7'-bis(phenylethynyl)-1,1'-biadamantane, while 3,3'-diethynyl-5,5',7,7'-tetramethyl-1,1'-biadamantane and 3,3',5,5'-tetraethynyl-1,1'-biadamantane are particularly preferred from the standpoint of solubility and heat resistance. Although diamantane compounds were mentioned as examples of polyamantane structures among the specific examples of compounds represented by general formula (1), there is no limitation to such compounds. Also, biadamantane compounds were mentioned as polyadamantane structures, but they may also be other polyadamantane compounds or poly(polyamantane) compounds wherein n is 2 or greater in general formula (1).

The groups represented by general formula (5) above will now be explained.

The groups represented by general formula (5) comprise hydrogen or an organic group as R₆. As organic groups for R₆ there may be mentioned the same ones as the organic groups for R₆ in general formula (3). In the groups represented by general formula (5), m2 is an integer of 1-5 and preferably 1-3.

Concrete examples of groups represented by general formula (5) above will now be explained.

As specific examples of acetylene bond-containing groups represented by general formula (5) above there may be mentioned those with hydrogen as R₆, such as 2-ethynylphenyl, 3-ethynylphenyl, 4-ethynylphenyl, 2,3-diethynylphenyl, 2,4-diethynylphenyl, 2,5-diethynylphenyl, 2,6-diethynylphenyl, 3,4-diethynylphenyl, 3,5-diethynylphenyl, 2,3,4-triethynylphenyl, 2,3,5-triethynylphenyl, 2,3,6-triethynylphenyl, 2,4,5-triethynylphenyl, 2,4,6-triethynylphenyl, 3,4,5-triethynylphenyl, 2,3,4,5-tetraethynylphenyl, 2,3,4,6-tetraethynylphenyl, 2,3,5,5-tetraethynylphenyl and 2,3,4,5,6-pentaethynylphenyl; those with methyl as R₆, such as 2-methylethynylphenyl, 3-methylethynylphenyl, 4-methylethynylphenyl, 2,3-dimethylethynylphenyl, 2,4-dimethylethynylphenyl, 2,5-dimethylethynylphenyl, 2,6-dimethylethynylphenyl, 3,4-dimethylethynylphenyl, 3,5-dimethylethynylphenyl, 2,3,4-trimethylethynylphenyl, 2,3,5-trimethylethynylphenyl, 2,3,6-trimethylethynylphenyl, 2,4,5-trimethylethynylphenyl, 2,4,6-trimethylethynylphenyl, 3,4,5-trimethylethynylphenyl, 2,3,4,5-tetramethylethynylphenyl, 2,3,4,6-tetramethylethynylphenyl, 2,3,5,6-tetramethylethynylphenyl and 2,3,4,5,6-pentamethylethynylphenyl; and those with phenyl as R₆, such as 2-phenylethynylphenyl, 3-phenylethynylphenyl, 4-phenylethynylphenyl, 2,3-diphenylethynylphenyl, 2,4-diphenylethynylphenyl, 2,5-diphenylethynylphenyl, 2,6-phenyldiethynylphenyl, 3,4-diphenylethynylphenyl, 3,5-diphenylethynylphenyl, 2,3,4-triphenylethynylphenyl, 2,3,5-triphenylethynylphenyl, 2,3,6-triphenylethynylphenyl, 2,4,5-triphenylethynylphenyl, 2,4,6-triphenylethynylphenyl, 3,4,5-triphenylethynylphenyl, 2,3,4,5-tetraphenylethynylphenyl, 2,3,4,6-tetraphenylethynylphenyl, 2,3,5,6-tetraphenylethynylphenyl and 2,3,4,5,6-pentaphenylethynylphenyl, with no limitation to these. Preferred among these are 4-ethynylphenyl, 3,5-diethynylphenyl, 3,4-diethynylphenyl, 4-methylethynylphenyl, 3,5-dimethylethynylphenyl and 3,4-dimethylethynylphenyl, from the viewpoint of ease of synthesis and solubility when used as a varnish. The hydrogens in the aforementioned organic groups may be replaced by fluorine atoms, methyl, trifluoromethyl, phenyl or the like.

As specific examples of acetylene bond-containing groups represented by general formula (5), among the compounds represented by general formula (1), there may be mentioned those with hydrogen as R₆, such as 4,9-bis(3,5-diethynylphenyl)diamantane, 2,4,7,9-tetrakis(3,5-diethynylphenyl)diamantane, 4,4'-bis(3,5-diethynylphenyl)-9,9'-bi(diamantane), 3,3'-bis(3,5-diethynylphenyl)-1,1'-biadamantane, 3,3',5,5'-tetramethyl-7,7'-bis(3,5-diethynylphenyl)-1,1'-biadamantane, 3,5-bis(3,5-diethynylphenyl)-1,1'-biadamantane, 3,5,3'-tris(3,5-diethynylphenyl)-1,1'-biadamantane, 3,3',5,5'-tetrakis(3,5-diethynylphenyl)-1,1'-biadamantane, 3,3',5,7-tetrakis(3,5-diethynylphenyl)-1,1'-biadamantane, 3,3',5,5',7-pentakis(3,5-diethynylphenyl)-1,1'-biadamantane, 3,3',5,5',7,7'-hexakis(3,5-diethynylphenyl)-1,1'-biadamantane, 3,3',5,5'-tetraphenyl-7,7'-bis(3,5-diethynylphenyl)-1,1'-biadamantane, 3,3'-bis(3,5-diethynylphenyl)-1,2'-biadamantane, 3,3'-bis(3,5-diethynylphenyl)-2,2'-biadamantane, 3,3',5,5'-tetramethyl-7,7'-bis(4-ethynylphenyl)-1,1'-biadamantane, 3,3'-bis(3,4-diethynylphenyl)-1,1'-biadamantane and 3,3'-bis(2,3,5-triethynylphenyl)-1,1'-biadamantane; those with methyl among the aforementioned organic groups as R₆, such as 4,9-bis(3,5-dimethylethynylphenyl)diamantane, 2,4,7,9-tetrakis(3,5-dimethylethynylphenyl)diamantane, 4,4'-bis(3,5-dimethylethynylphenyl)-9,9'-bi(diamantane), 3,3'-bis(3,5-dimethylethynylphenyl)-1,1'-biadamantane, 3,3',5,5'-tetramethyl-7,7'-bis(3,5-dimethylethynylphenyl)-1,1'-biadamantane, 3,5-bis(3,5-dimethylethynylphenyl)-1,1'-biadamantane, 3,5,3'-tris(3,5-dimethylethynylphenyl)-1,1'-biadamantane, 3,3',5,5'-tetrakis(3,5-dimethylethynylphenyl)-1,1'-biadamantane, 3,3',5,7-tetrakis(3,5-dimethylethynylphenyl)-1,1'-biadamantane, 3,3',5,5',7-pentakis(3,5-dimethylethynylphenyl)-1,1'-biadamantane, 3,3',5,5',7,7'-hexakis(3,5-dimethylethynylphenyl)-1,1'-biadamantane, 3,3',5,5'-tetraphenyl-7,7'-bis(3,5-dimethylethynylphenyl)-1,1'-biadamantane, 3,3'-bis(3,5-dimethylethynylphenyl)-1,2'-biadamantane, 3,3'-bis(3,5-dimethylethynylphenyl)-2,2'-biadamantane, 3,3',5,5'-tetramethyl-7,7'-bis(4-methylethynylphenyl)-1,1'-biadamantane, 3,3'-bis(3,4-dimethylethynylphenyl)-1,1'-biadamantane and 3,3'-bis(2,3,5-trimethylethynylphenyl)-1,1'-biadamantane; and those with phenyl among the aforementioned organic groups as R₆, such as 4,9-bis(3,5-diphenylethynylphenyl)diamantane, 2,4,7,9-tetrakis(3,5-diphenylethynylphenyl)diamantane, 4,4'-bis(3,5-diphenylethynylphenyl)-9,9'-bi(diamantane), 3,3'-bis(3,5-diphenylethynylphenyl)-1,1'-biadamantane, 3,3',5,5'-tetramethyl-7,7'-bis(3,5-diphenylethynylphenyl)-1,1'-biadamantane, 3,5-bis(3,5-diphenylethynylphenyl)-1,1'-biadamantane, 3,5,3'-tris(3,5-diphenylethynylphenyl)-1,1'-biadamantane, 3,3',5,5'-tetrakis(3,5-diphenylethynylphenyl)-1,1'-biadamantane, 3,3',5,7-tetrakis(3,5-diphenylethynylphenyl)-1,1'-biadamantane, 3,3',5,5',7-pentakis(3,5-diphenylethynylphenyl)-1,1'-biadamantane, 3,3',5,5',7,7'-hexakis(3,5-diphenylethynylphenyl)-1,1'-biadamantane, 3,3',5,5'-tetraphenyl-7,7'-bis(3,5-diphenylethynylphenyl)-1,1'-biadamantane, 3,3'-bis(3,5-diphenylethynylphenyl)-1,2'-biadamantane, 3,3'-bis(3,5-diphenylethynylphenyl)-2,2'-biadamantane, 3,3'-bis(3,4-diphenylethynylphenyl)-1,1'-biadamantane and 3,3'-bis(2,3,5-triphenylethynylphenyl)-1,1'-biadamantane, with no limitation to these. Preferred among these are 3,3',5,5'-tetramethyl-7,7'-bis(4-ethynylphenyl)-1,1'-biadamantane, 3,3'-bis(3,4-diethynylphenyl)-1,1'-biadamantane, 3,3',5,5'-tetramethyl-7,7'-bis(3,5-diethynylphenyl)-1,1'-biadamantane, 3,3',5,5'-tetramethyl-7,7'-bis(4-methylethynylphenyl)-1,1'-biadamantane, 3,3'-bis(3,4-dimethylethynylphenyl)-1,1'-biadamantane, 3,3',5,5'-tetramethyl-7,7'-bis(3,5-dimethylethynylphenyl)-1,1'-biadamantane, 3,3',5,5'-tetramethyl-7,7'-bis(3,5-diphenylethynylphenyl)-1,1'-biadamantane and the like, and especially preferred are 3,3',5,5'-tetramethyl-7,7'-bis(3,5-diethynylphenyl)-1,1'-biadamantane and 3,3',5,5'-tetramethyl-7,7'-bis(3,5-diphenylethynylphenyl)-1,1'-biadamantane from the viewpoint of solubility and heat resistance. Although diamantane compounds were mentioned as examples of polyamantane structures among the specific examples of compounds represented by general formula (1), there is no limitation to such compounds. Also, biadamantane compounds were mentioned as polyadamantane structures, but they may also be other polyadamantane compounds or poly(polyamantane) compounds wherein n is 2 or greater in general formula (1).

The groups represented by general formula (6) above will now be explained.

The groups represented by general formula (6) comprise a single bond or an aromatic group as Z. As aromatic groups for Z there may be mentioned the same aromatic groups for Z in the groups represented by general formula (3).

The groups represented by general formula (6) also comprise an aliphatic group as R₅. As aliphatic groups for R₅ there may be mentioned the same ones as the aliphatic groups for R₅ in the groups represented by general formula (3).

Specifically, in the groups represented by general formula (6), R₆, R₇ and R₈ are each independently hydrogen or an organic group. As organic groups for R₆, R₇ and R₈ there may be mentioned the same ones as the organic groups for R₆ in the groups represented by general formula (3). When Z in the group represented by general formula (6) is a single bond m will be 1, and when Z is an aromatic group m1 will be 1 or 2.

Concrete examples of groups represented by general formula (6) above will now be explained.

As specific examples of vinyl bond-containing groups represented by general formula (6), there may be mentioned alkenyloxy, alkenyloxyphenyl and bis(alkenyloxy)phenyl. As examples of alkenyloxy groups there may be mentioned 2-propenyloxy, 2-butenyloxy, 3-butenyloxy, 2-hexenyloxy, 3-hexenyloxy, 4-hexenyloxy, 5-hexenyloxy, 2-heptenyloxy, 3-heptenyloxy, 4-heptenyloxy, 5-heptenyloxy, 6-heptenyloxy, 1,1-dimethyl-2-propenyloxy, 1,1-diphenyl-2-propenyloxy, 2,3-dimethyl-2-butenyloxy group and 2,3-diphenyl-2-butenyloxy, as examples of alkenyloxyphenyl groups there may be mentioned 4-(2-propenyloxy)phenyl, 4-(2-butenyloxy)phenyl, 4-(3-butenyloxy)phenyl, 4-(2-hexenyloxy)phenyl, 4-(3-hexenyloxy)phenyl, 4-(4-hexenyloxy)phenyl, 4-(5-hexenyloxy)phenyl, 4-(2-heptenyloxy)phenyl, 4-(3-heptenyloxy)phenyl, 4-(4-heptenyloxy)phenyl, 4-(5-heptenyloxy)phenyl, 4-(6-heptenyloxy)phenyl, 4-(1,1-dimethyl-2-propenyloxy)phenyl, 4-(1,1-diphenyl-2-propenyloxy)phenyl, 4-(2,3-dimethyl-2-butenyloxy)phenyl group and 4-(2,3-diphenyl-2-butenyloxy)phenyl, and as examples of bis(alkynyloxy)phenyl groups there may be mentioned 2,4-bis(2-propenyloxy)phenyl, 2,4-bis(2-butenyloxy)phenyl, 2,4-bis(3-butenyloxy)phenyl, 2,4-bis(2-hexenyloxy)phenyl, 2,4-bis(3-hexenyloxy)phenyl, 2,4-bis(4-hexenyloxy)phenyl, 2,4-bis(5-hexenyloxy)phenyl, 2,4-bis(2-heptenyloxy)phenyl, 2,4-bis(3-heptenyloxy)phenyl, 2,4-bis(4-heptenyloxy)phenyl, 2,4-bis(5-heptenyloxy)phenyl, 2,4-bis(6-heptenyloxy)phenyl, 2,4-bis(1,1-dimethyl-2-propenyloxy)phenyl, 2,4-bis(1,1-diphenyl-2-propenyloxy)phenyl, 2,3-bis(2-propenyloxy)phenyl, 2,5-bis(2-propenyloxy)phenyl, 2,6-bis(2-propenyloxy)phenyl, 3,4-bis(2-propenyloxy)phenyl, 3,5-bis(2-propenyloxy)phenyl and 3,5-bis(2,3-dimethyl-2-butenyloxy)phenyl, with no limitation to these. The hydrogens in the aforementioned vinyl bond-containing groups may be replaced by fluorine atoms, methyl, trifluoromethyl, phenyl or the like.

Among these there are preferred 2-propenyloxy, (2-propenyloxy)phenyl and bis(2-propenyloxy)phenyl, and more preferably 2-propenyloxy, 4-(2-propenyloxy)phenyl and 2,4-bis(2-propenyloxy)phenyl, in order to obtain organic insulating films with more excellent heat resistance.

As specific examples of vinyl bond-containing groups represented by general formula (6), among the compounds represented by general formula (1), there may be mentioned those with alkenyloxy groups, specifically 2-propenyloxy groups, such as 4,9-bis(2-propenyloxy)diamantane, 2,4,7,9-tetrakis(2-propenyloxy)diamantane, 4,4'-bis(2-propenyloxy)-9,9'-bi(diamantane), 3,3'-bis(2-propenyloxy)-1,1'-biadamantane, 3,3',5,5'-tetramethyl-7,7'-bis(2-propenyloxy)-1,1'-biadamantane, 3,5-bis(2-propenyloxy)-1,1'-biadamantane, 3,5,3'-tris(2-propenyloxy)-1,1'-biadamantane, 3,3',5,5'-tetrakis(2-propenyloxy)-1,1'-biadamantane, 3,3',5,7-tetrakis(2-propenyloxy)-1,1'-biadamantane, 3,3',5,5',7-pentakis(2-propenyloxy)-1,1'-biadamantane, 3,3',5,5',7,7'-hexakis(2-propenyloxy)-1,1'-biadamantane, 3,3',5,5'-tetramethyl-7,7'-bis(2-propenyloxy)-1,1'-biadamantane, 3,3',5,5'-tetraphenyl-7,7'-bis(2-propenyloxy)-1,1'-biadamantane, 3,3'-bis(2-propenyloxy)-1,2'-biadamantane, 3,3'-bis(2-propenyloxy)-2,2'-biadamantane, 3,3'-bis(2-propenyloxy)-1,2'-biadamantane and 3,3'-bis(2-methyl-2-propenyloxy)-2,2'-biadamantane; those with alkenyloxyphenyl groups, specifically 2-propenyloxyphenyl groups, such as 4,9-bis(4-(2-propenyloxy)phenyl)diamantane, 2,4,7,9-tetrakis(4-(2-propenyloxy)phenyl)diamantane, 4,4'-bis(4-(2-propenyloxy)phenyl)-9,9'-bi(diamantane), 3,3'-bis(4-(2-propenyloxy)phenyl)-1,1'-biadamantane, 3,3',5,5'-tetramethyl-7,7'-bis(4-(2-propenyloxy)phenyl)-1,1'-biadamantane, 3,5-bis(4-(2-propenyloxy)phenyl)-1,1'-biadamantane, 3,5,3'-tris(4-(2-propenyloxy)phenyl)-1,1'-biadamantane, 3,3',5,5'-tetrakis(4-(2-propenyloxy)phenyl)-1,1'-biadamantane, 3,3',5,7-tetrakis(4-(2-propenyloxy)phenyl)-1,1'-biadamantane, 3,3',5,5',7-pentakis(4-(2-propenyloxy)phenyl)-1,1'-biadamantane, 3,3',5,5',7,7'-hexakis(4-(2-propenyloxy)phenyl)-1,1'-biadamantane, 3,3',5,5'-tetramethyl-7,7'-bis(9-(2-propenyloxy)phenyl)-1,1'-biadamantane, 3,3',5,5'-tetraphenyl-7,7'-bis(4-(2-propenyloxy)phenyl)-1,1'-biadamantane, 3,3'-bis(4-(2-propenyloxy)phenyl)-1,2'-biadamantane, 3,3'-bis(4-(2-propenyloxy)phenyl)-2,2'-biadamantane and 3,3'-bis(4-(2-methyl-2-propenyloxy)phenyl)-2,2'-biadamantane; and those with bis(alkenyloxy)phenyl groups, specifically bis(2-propenyloxy)phenyl groups, such as 4,9-bis(2,4-bis(2-propenyloxy)phenyl)diamantane, 2,4,7,9-tetrakis(2,4-bis(2-propenyloxy)phenyl)diamantane, 4,4'-bis(2,4-bis(2-propenyloxy)phenyl-9,9'-bi(diamantane), 3,3'-bis(2,4-bis(2-propenyloxy)phenyl)-1,1'-biadamantane, 3,3',5,5'-tetramethyl-7,7'-bis(2,4-bis(2-propenyloxy)phenyl)-1,1'-biadamantane, 3,5-bis(2,4-bis(2-propenyloxy)phenyl)-1,1'-biadamantane, 3,5,3'-tris(2,4-bis(2-propenyloxy)phenyl)-1,1'-biadamantane, 3,3',5,5'-tetrakis(2,4-bis(2-propenyloxy)phenyl)-1,1'-biadamantane, 3,3',5,7-tetrakis(2,4-bis(2-propenyloxy)phenyl)-1,1'-biadamantane, 3,3',5,5',7-pentakis(2,4-bis(2-propenyloxy)phenyl)-1,1'-biadamantane, 3,3',5,5',7,7'-hexakis(2,4-bis(2-propenyloxy)phenyl)-1,1'-biadamantane, 3,3',5,5'-tetramethyl-7,7'-bis(2,4-bis(2-propenyloxy)phenyl)-1,1'-biadamantane, 3,3',5,5'-tetraphenyl-7,7'-bis(2,4-bis(2-propenyloxy)phenyl)-1,1'-biadamantane, 3,3'-bis(2,4-bis(2-propenyloxy)phenyl)-1,2'-biadamantane, 3,3'-bis(2,4-bis(2-propenyloxy)phenyl)-2,2'-biadamantane and 3,3'-bis(2,4-bis(2-methyl-2-propenyloxy)phenyl)-2,2'-biadamantane, with no limitation to these. Preferred among these are 3,3',5,5'-tetramethyl-7,7'-bis(2-propenyloxy)-1,1-biadamantane, 3,3',5,5'-tetramethyl-7,7'-bis(4-(2-propenyloxy)phenyl)-1,1'-biadamantane and 3,3',5,5'-tetramethyl-7,7'-bis(2,4-bis(2-propenyloxy)phenyl)-1,1'-biadamantane, with 3,3',5,5'-tetramethyl-7,7'-bis(2-propenyloxy)-1,1-biadamantane being particularly preferred. Although diamantane compounds were mentioned as examples of polyamantane structures among the specific examples of compounds represented by general formula (1), there is no limitation to such compounds. Also, biadamantane compounds were mentioned as polyadamantane structures, but they may also be other polyadamantane compounds or poly(polyamantane) compounds wherein n is 2 or greater in general formula (1).

The groups represented by general formula (7) above will now be explained.

Specifically, in the groups represented by general formula (7), R₆, R₇ and R₈ are each independently hydrogen or an organic group. As organic groups for R₆, R₇ and R₈ there may be mentioned the same ones as the organic groups for R₆ in the groups represented by general formula (3).

Concrete examples of groups represented by general formula (7) above will now be explained.

As specific examples of vinyl bond-containing groups represented by general formula (7) there may be mentioned vinyl, 1-phenylethenyl, 2-phenylethenyl, 1,1-diphenylethenyl, 1,2-diphenylethenyl, 1,2,2-triphenylethenyl, propenyl, isopropenyl, 1-methylpropenyl, 2-methylpropenyl, 1,2-dimethylpropenyl, 1-phenylpropenyl, 2-phenylpropenyl, 1,2-diphenylpropenyl, 2-phenylisopropenyl, 2,2-diphenylisopropenyl, 1-phenyl-2-methylpropenyl, 2-phenyl-1-methylpropenyl and 2-phenyl-2-methylpropenyl, among which vinyl, 2-phenylethenyl and propenyl groups are preferred from the viewpoint of heat resistance for use in organic insulating films. The examples given here are not specified as *cis* or *trans* forms, and either are suitable. The hydrogens in the aforementioned vinyl bond-containing groups may be replaced by fluorine atoms, methyl, trifluoromethyl or phenyl.

As specific examples of vinyl bond-containing groups represented by general formula (7), among the compounds represented by general formula (1), there may be mentioned those with vinyl groups as vinyl bond-containing groups represented by general formula (7), such as 4,9-divinyldiamantane, 2,4,7,9-tetradivinyldiamantane, 4,4'-divinyl-9,9'-bi(diamantane), 3,3'-divinyl-1,1'-biadamantane, 3,3',5,5'-tetramethyl-7,7'-divinyl-1,1'-biadamantane, 3,5-divinyl-1,1'-biadamantane, 3,5,3'-trivinyl-1,1'-biadamantane, 3,3',5,5'-tetravinyl-1,1'-biadamantane, 3,3',5,7-tetravinyl-1,1'-biadamantane, 3,3',5,5',7-pentavinyl-1,1'-biadamantane, 3,3',5,5',7,7'-hexavinyl-1,1'-biadamantane, 3,3',5,5'-tetraphenyl-7,7'-divinyl-1,1'-biadamantane, 3,3'-divinyl-1,2'-biadamantane and 3,3'-divinyl-2,2'-biadamantane; those with 2-phenylethenyl groups as vinyl bond-containing groups represented by general formula (7), such as 4,9-bis(2-phenylethenyl)diamantane, 2,4,7,9-tetrakis(2-phenylethenyl)diamantane, 4,4'-bis(2-phenylethenyl)-9,9'-bi(diamantane), 3,3'-bis(2-phenylethenyl)-1,1'-biadamantane, 3,3',5,5'-tetramethyl-7,7'-bis(2-phenylethenyl)-1,1'-biadamantane, 3,5-bis(2-phenylethenyl)-1,1'-biadamantane, 3,5,3'-tris(2-phenylethenyl)-1,1'-biadamantane, 3,3',5,5'-tetrakis(2-phenylethenyl)-1,1'-biadamantane, 3,3',5,7-tetrakis(2-phenylethenyl)-1,1'-biadamantane, 3,3',5,5',7-pentakis(2-phenylethenyl)-1,1'-biadamantane, 3,3',5,5',7,7'-hexakis(2-phenylethenyl)-1,1'-biadamantane, 3,3',5,5'-tetraphenyl-7,7'-bis(2-phenylethenyl)-1,1'-biadamantane, 3,3'-bis(2-phenylethenyl)-1,2'-biadamantane and 3,3'-bis(2-phenylethenyl)-2,2'-biadamantane; and those with propenyl groups as vinyl bond-containing groups represented by general formula (7), such as 4,9-dipropenyldiamantane, 2,4,7,9-tetrapropenyldiamantane, 4,4'-dipropenyl-9,9'-bi(diamantane), 3,3'-dipropenyl-1,1'-biadamantane, 3,3',5,5'-tetramethyl-7,7'-dipropenyl-1,1'-biadamantane, 3,5-dipropenyl-1,1'-biadamantane, 3,5,3'-tripropenyl-1,1'-biadamantane, 3,3',5,5'-tetrapropenyl-1,1'-biadamantane, 3,3',5,7-tetrapropenyl-1,1'-biadamantane, 3,3',5,5',7-pentapropenyl-1,1'-biadamantane, 3,3',5,5',7,7'-hexapropenyl-1,1'-biadamantane, 3,3',5,5'-tetraphenyl-7,7'-dipropenyl-1,1'-biadamantane, 3,3'-dipropenyl-1,2'-biadamantane and 3,3'-dipropenyl-2,2'-biadamantane. Preferred among these are 3,3'-divinyl-5,5',7,7'-tetramethyl-1,1'-biadamantane, 3,3',5,5'-tetravinyl-1,1'-biadamantane, 3,3',5,5'-tetramethyl-7,7'-bis(2-phenylethenyl)-1,1'-biadamantane and 3,3',5,5'-tetramethyl-7,7'-dipropenyl-1,1'-biadamantane, with 3,3'-divinyl-5,5',7,7'-tetramethyl-1,1'-biadamantane and 3,3',5,5'-tetravinyl-1,1'-biadamantane being particularly preferred from the viewpoint of solubility and heat resistance, and with no limitation to these. Although diamantane compounds were mentioned as examples of polyamantane structures among the specific examples of compounds represented by general formula (1), there is no limitation to such compounds. Also, biadamantane compounds were mentioned as polyadamantane structures, but they may also be other polyadamantane compounds or poly(polyamantane) compounds wherein n is 2 or greater in general formula (1).

The groups represented by general formula (8) above will now be explained.

Specifically, in the groups represented by general formula (8), R₆, R₇ and R₈ are each independently hydrogen or an organic group. As organic groups for R₆, R₇ and R₈ there may be mentioned the same ones as the organic groups for R₆ in the groups represented by general formula (3). In the groups represented by general formula (8), m2 is an integer of 1-5 and preferably 1-3.

Concrete examples of groups represented by general formula (8) above will now be explained.

As specific examples of vinyl bond-containing groups represented by general formula (8) there may be mentioned those with vinylphenyl groups as vinyl bond-containing groups represented by general formula (8), such as 2-vinylphenyl, 3-vinylphenyl, 4-vinylphenyl, 2,3-divinylphenyl, 2,4-divinylphenyl, 2,5-divinylphenyl, 2,6-divinylphenyl, 3,4-divinylphenyl, 3,5-divinylphenyl, 2,3,4-trivinylphenyl, 2,3,5-trivinylphenyl, 2,3,6-trivinylphenyl, 2,4,5-trivinylphenyl, 2,4,6-trivinylphenyl, 3,4,5-trivinylphenyl, 2,3,4,5-tetravinylphenyl, 2,3,4,6-tetravinylphenyl, 2,3,5,6-tetravinylphenyl and 2,3,4,5,6-pentavinylphenyl; those with 2-phenylethenylphenyl groups as vinyl bond-containing groups represented by general formula (8), such as 2-(2-phenylethenyl)phenyl, 3-(2-phenylethenyl)phenyl, 4-(2-phenylethenyl)phenyl, 2,3-bis(2-phenylethenyl)phenyl, 2,4-bis(2-phenylethenyl)phenyl, 2,5-bis(2-phenylethenyl)phenyl, 2,6-bis(2-phenylethenyl)phenyl, 3,4-bis(2-phenylethenyl)phenyl, 3,5-bis(2-phenylethenyl)phenyl, 2,3,4-tris(2-phenylethenyl)phenyl, 2,3,5-tris(2-phenylethenyl)phenyl, 2,3,6-tris(2-phenylethenyl)phenyl, 2,4,5-tris(2-phenylethenyl)phenyl, 2,4,6-tris(2-phenylethenyl)phenyl, 3,4,5-tris(2-phenylethenyl)phenyl, 2,3,4,5-tetrakis(2-phenylethenyl)phenyl, 2,3,4,6-tetrakis(2-phenylethenyl)phenyl, 2,3,5,6-tetrakis(2-phenylethenyl)phenyl and 2,3,4,5,6-pentakis(2-phenylethenyl)phenyl; and those with propenylphenyl groups as vinyl bond-containing groups represented by general formula (8), such as 2-propenylphenyl, 3-propenylphenyl, 4-propenylphenyl, 2,3-dipropenylphenyl, 2,4-dipropenylphenyl, 2,5-dipropenylphenyl, 2,6-dipropenylphenyl, 3,4-dipropenylphenyl, 3,5-dipropenylphenyl, 2,3,4-tripropenylphenyl, 2,3,5-tripropenylphenyl, 2,3,6-tripropenylphenyl, 2,4,5-tripropenylphenyl, 2,4,6-tripropenylphenyl, 3,4,5-tripropenylphenyl, 2,3,4,5-tetrapropenylphenyl, 2,3,4,6-tetrapropenylphenyl, 2,3,5,6-tetrapropenylphenyl and 2,3,4,5,6-pentapropenylphenyl, with no limitation to these. Preferred among these are 4-vinylphenyl, 3,5-divinylphenyl, 3,4-divinylphenyl, 4-propenylphenyl, 3,5-dipropenylphenyl and 3,4-dipropenylphenyl, from the viewpoint of ease of synthesis and solubility when used in a varnish. The hydrogens in the aforementioned vinyl bond-containing groups may be replaced by fluorine atoms, methyl, trifluoromethyl or phenyl.

As specific examples with vinyl bond-containing groups represented by general formula (8), among the compounds represented by general formula (1), there may be mentioned vinyl bond-containing groups, specifically vinylphenyl groups, represented by general formula (8) such as 4,9-bis(3,5-divinylphenyl)diamantane, 2,4,7,9-tetrakis(3,5-divinylphenyl)diamantane, 4,4'-bis(3,5-divinylphenyl)-9,9'-bi(diamantane), 3,3'-bis(3,5-divinylphenyl)-1,1'-biadamantane, 3,3',5,5'-tetramethyl-7,7'-bis(3,5-divinylphenyl)-1,1'-biadamantane, 3,5-bis(3,5-divinylphenyl)-1,1'-biadamantane, 3,5,3'-tris(3,5-divinylphenyl)-1,1'-biadamantane, 3,3',5,5'-tetrakis(3,5-divinylphenyl)-1,1'-biadamantane, 3,3',5,7-tetrakis(3,5-divinylphenyl)-1,1'-biadamantane, 3,3',5,5',7-pentakis(3,5-divinylphenyl)-1,1'-biadamantane, 3,3',5,5',7,7'-hexakis(3,5-divinylphenyl)-1,1'-biadamantane, 3,3',5,5'-tetraphenyl-7,7'-bis(3,5-divinylphenyl)-1,1'-biadamantane, 3,3'-bis(3,5-divinylphenyl)-1,2'-biadamantane, 3,3'-bis(3,5-divinylphenyl)-2,2'-biadamantane, 3,3'-bis(3,4-divinylphenyl)-1,1'-biadamantane and 3,3'-bis(2,3,5-trivinylphenyl)-1,1'-biadamantane; vinyl bond-containing groups, specifically 2-phenylethenylphenyl groups, represented by general formula (8) such as 4,9-bis(3,5-bis(2-phenylethenyl)phenyl)diamantane, 2,4,7,9-tetrakis(3,5-bis(2-phenylethenyl)phenyl)diamantane, 4,4'-bis(3,5-bis(2-phenylethenyl)phenyl)-9,9'-bi(diamantane), 3,3'-bis(3,5-bis(2-phenylethenyl)phenyl)-1,1'-biadamantane, 3,3',5,5'-tetramethyl-7,7'-bis(3,5-bis(2-phenylethenyl)phenyl)-1,1'-biadamantane, 3,5-bis(3,5-bis(2-phenylethenyl)phenyl)-1,1'-biadamantane, 3,5,3'-tris(3,5-bis(2-phenylethenyl)phenyl)-1,1'-biadamantane, 3,3',5,5'-tetrakis(3,5-bis(2-phenylethenyl)phenyl)-1,1'-biadamantane, 3,3',5,7-tetrakis(3,5-bis(2-phenylethenyl)phenyl)-1,1'-biadamantane, 3,3',5,5',7-pentakis(3,5-bis(2-phenylethenyl)phenyl)-1,1'-biadamantane, 3,3',5,5',7,7'-hexakis(3,5-bis(2-phenylethenyl)phenyl)-1,1'-biadamantane, 3,3',5,5'-tetraphenyl-7,7'-bis(3,5-bis(2-phenylethenyl)phenyl)-1,1'-biadamantane, 3,3'-bis(3,5-bis(2-phenylethenyl)phenyl)-1,2'-biadamantane, 3,3'-bis(3,5-bis(2-phenylethenyl)phenyl)-2,2'-biadamantane, 3,3'-bis(3,4-bis(2-phenylethenyl)phenyl)-1,1'-biadamantane and 3,3'-bis(2,3,5-tris(2-phenylethenyl)phenyl)-1,1'-biadamantane; and vinyl bond-containing groups, specifically propenylphenyl groups, represented by general formula (8) such as 4,9-bis(3,5-dipropenylphenyl)diamantane, 2,4,7,9-tetrakis(3,5-dipropenylphenyl)diamantane, 4,4'-bis(3,5-dipropenylphenyl)-9,9'-bi(diamantane), 3,3'-bis(3,5-dipropenylphenyl)-1,1'-biadamantane, 3,3',5,5'-tetramethyl-7,7'-bis(3,5-dipropenylphenyl)-1,1'-biadamantane, 3,5-bis(3,5-dipropenylphenyl)-1,1'-biadamantane, 3,5,3'-tris(3,5-dipropenylphenyl)-1,1'-biadamantane, 3,3',5,5'-tetrakis(3,5-dipropenylphenyl)-1,1'-biadamantane, 3,3',5,7-tetrakis(3,5-dipropenylphenyl)-1,1'-biadamantane, 3,3',5,5',7-pentakis(3,5-dipropenylphenyl)-1,1'-biadamantane, 3,3',5,5',7,7'-hexakis(3,5-dipropenylphenyl)-1,1'-biadamantane, 3,3',5,5'-tetraphenyl-7,7'-bis(3,5-dipropenylphenyl)-1,1'-biadamantane, 3,3'-bis(3,5-dipropenylphenyl)-1,2'-biadamantane, 3,3'-bis(3,5-dipropenylphenyl)-2,2'-biadamantane, 3,3'-bis(3,4-dipropenylphenyl)-1,1'-biadamantane and 3,3'-bis(2,3,5-tripropenylphenyl)-1,1'-biadamantane, with no limitation to these. Preferred among these are 3,3',5,5'-tetramethyl-7,7'-bis(3,5-divinylphenyl)-1,1'-biadamantane, 3,3',5,5'-tetramethyl-7,7'-bis(3,5-bis(2-phenylethenyl)phenyl)-1,1'-biadamantane and 3,3',5,5'-tetramethyl-7,7'-bis(3,5-dipropenylphenyl)-1,1'-biadamantane, and particularly preferred is 3,3',5,5'-tetramethyl-7,7'-bis(3,5-divinylphenyl)-1,1'-biadamantane from the viewpoint of solubility and heat resistance. Although diamantane compounds were mentioned as examples of polyamantane structures among the specific examples of compounds represented by general formula (1), there is no limitation to such compounds. Also, biadamantane compounds were mentioned as polyadamantane structures, but they may also be other polyadamantane compounds or poly(polyamantane) compounds wherein n is 2 or greater in general formula (1).

Typical processes for production of compounds represented by general formula (1) above will now be described.

As an example of a process for production of compounds with acetylene bond-containing groups represented by general formula (3), of the compounds represented by general formula (1), there may be mentioned a synthesis process involving nucleophilic substitution reaction of a halogenated alkynyl compound and a hydroxybiadamantane compound or hydroxyaromatic biadamantane compound, in the presence of a base such as sodium hydroxide. In this case the compound represented by general formula (1) is a biadamantane compound, but the same description applies for other polyadamantane compounds wherein n is 2 or greater, as well as polyamantane compounds or poly(polyamantane) compounds, of the compounds represented by general formula (1).

As examples of halogenated alkynyl compounds there may be mentioned 3-bromo-1-propyne, 4-bromo-2-butyne, 4-bromo-3-butyne, 6-bromo-2-hexyne, 6-bromo-3-hexyne, 1-bromo-3-hexyne, 1-bromo-2-hexyne, 7-bromo-2-heptyne, 7-bromo-3-heptyne, 1-bromo-4-heptyne, 1-bromo-3-heptyne, 1-bromo-2-heptyne, 3-bromo-3,3-dimethyl-1-propyne, 3-bromo-3,3-diphenyl-1-propyne, 3-iodo-1-propyne and 3-chloro-1-propyne, with no limitation to these.

As examples of hydroxybiadamantane compounds there may be mentioned 3,3'-dihydroxy-1,1'-biadamantane, 3,3'-dihydroxy-5,5',7,7'-tetramethyl-1,1'-biadamantane, 3,5-dihydroxy-1,1'-biadamantane, 3,5,3'-trihydroxy-1,1'-biadamantane, 3,3',5,5'-tetrahydroxy-1,1'-biadamantane, 3,3',5,7-tetrahydroxy-1,1'-biadamantane, 3,3',5,5',7-pentahydroxy-1,1'-biadamantane, 3,3',5,5',7,7'-hexahydroxy-1,1'-biadamantane, 3,3',5,5'-tetramethyl-7,7'-dihydroxy-1,1'-biadamantane, 3,3',5,5'-tetraphenyl-7,7'-dihydroxy-1,1'-biadamantane, 3,3'-dihydroxy-1,2'-biadamantane and 3,3'-dihydroxy-2,2'-biadamantane, with no limitation to these.

As examples of hydroxyaromatic biadamantane compounds there may be mentioned 3,3'-bis(4-hydroxyphenyl)-1,1'-biadamantane, 3,3'-bis(4-hydroxyphenyl)-5,5',7,7'-tetramethyl-1,1'-biadamantane, 3,5-bis(4-hydroxyphenyl)-1,1'-biadamantane, 3,5,3'-tris(4-hydroxyphenyl)-1,1'-biadamantane, 3,3',5,5'-tetrakis(4-hydroxyphenyl)-1,1'-biadamantane, 3,3',5,7-tetrakis(4-hydroxyphenyl)-1,1'-biadamantane, 3,3',5,5',7-pentakis(4-hydroxyphenyl)-1,1'-biadamantane, 3,3',5,5',7,7'-hexakis(4-hydroxyphenyl)-1,1'-biadamantane, 3,3',5,5'-tetramethyl-7,7'-bis(4-hydroxyphenyl)-1,1'-biadamantane, 3,3',5,5'-tetraphenyl-7,7'-bis(4-hydroxyphenyl)-1,1'-biadamantane, 3,3'-bis(4-hydroxyphenyl)-1,2'-biadamantane, 3,3'-bis(4-hydroxyphenyl)-2,2'-biadamantane, 3,3'-bis(2,4-dihydroxyphenyl)-1,1'-biadamantane, 3,3'-bis(2,4-dihydroxyphenyl)-5,5',7,7'-tetramethyl-1,1'-biadamantane, 3,5-bis(2,4-dihydroxyphenyl)-1,1'-biadamantane, 3,5,3'-tris(2,4-dihydroxyphenyl)-1,1'-biadamantane, 3,3',5,5'-tetrakis(2,4-dihydroxyphenyl)-1,1'-biadamantane, 3,3',5,7-tetrakis(2,4-dihydroxyphenyl)-1,1'-biadamantane, 3,3',5,5',7-pentakis(2,4-dihydroxyphenyl)-1,1'-biadamantane, 3,3',5,5',7,7'-hexakis(2,4-dihydroxyphenyl)-1,1'-biadamantane, 3,3',5,5'-tetramethyl-7,7'-bis(2,4-dihydroxyphenyl)-1,1'-biadamantane, 3,3',5,5'-tetraphenyl-7,7'-bis(2,4-dihydroxyphenyl)-1,1'-biadamantane, 3,3'-bis(2,4-dihydxoxyphenyl)-1,2'-biadamantane and 3,3'-bis(2,4-dihydroxyphenyl)-2,2'-biadamantane, with no limitation to these.

The process for producing the compound with an acetylene bond-containing group with hydrogen as R₆ in the group represented by general formula (4), of the compounds represented by general formula (1), may be the process described in Macromolecules 1991, 24, 5261-5265, for example. In this case, the compound represented by general formula (1) is a biadamantane compound, but the same description applies for other polyadamantane compounds wherein n is 2 or greater, as well as polyamantane compounds or poly(polyamantane) compounds, of the compounds represented by general formula (1).

Specifically, first a halogenated biadamantane compound and bromoethene may be dissolved or dispersed in an appropriate solvent (dichloromethane or the like) and mixed for reaction to produce 2,2-dibromoethylbiadamantane. An appropriate catalyst (aluminum chloride or the like) that promotes reaction may be used for this step. The previously obtained 2,2-dibromoethylbiadamantane compound may then be dissolved or dispersed in an appropriate solvent (dimethyl sulfoxide or the like), a base such as potassium tert-butoxide added and mixed therewith for reaction, and then water added to produce a biadamantane compound having an acetylene bond-containing group with hydrogen as R₆ in the group represented by general formula (4).

As examples of halogenated biadamantane compounds there may be mentioned 3,3¹-dibromo-1,1'-biadamantane, 3,3'-dibromo-5,5',7,7'-tetramethyl-1,1'-biadamantane, 3,5-dibromo-1,1'-biadamantane, 3,5,3'-tribromo-1,1'-biadamantane, 3,3',5,5'-tetrabromo-1,1'-biadamantane, 3,3',5,7-tetrabromo-1,1'-biadamantane, 3,3',5,5',7-pentabromo-1,1'-biadamantane, 3,3',5,5',7,7'-hexabromo-1,1'-biadamantane, 3,3',5,5'-tetramethyl-7,7'-dibromo-1,1'-biadamantane, 3,3',5,5'-tetraphenyl-7,7'-dibromo-1,1'-biadamantane, 3,3'-dibromo-1,2'-biadamantane and 3,3'-dibromo-2,2'-biadamantane, with no limitation to these.

By reacting a halogenated alkane with a biadamantane compound having an acetylene bond-containing group with hydrogen as R₆ in the group represented by general formula (4), it is possible to produce a compound with an acetylene bond-containing group having an organic group as R₆ in a group represented by general formula (4), of the biadamantane compound structure represented by general formula (1). In order to promote the reaction, an appropriate catalyst such as copper(II) iodide, triphenylphosphine or dichlorobis(triphenylphosphine)palladium may be added.

The compound represented by general formula (1) is a biadamantane compound in this case as well, but the same description applies for other polyadamantane compounds wherein n is 2 or greater, as well as polyamantane compounds or poly(polyamantane) compounds, of the compounds represented by general formula (1).

As examples of halogenated alkanes there may be mentioned methane iodide, bromoethane, bromopropane, bromobutane, bromopentane, bromohexane, bromoheptane, bromooctane, bromoadamantane, 1-bromocycloheptane, 1-bromocyclohexane, 1-bromo crown ether, bromobenzene, 1-phenoxy-4-bromobenzene, bromonaphthalene, bromofluorene, 4-(methylethynyl)bromobenzene and 4-(phenylethynyl)bromobenzene, among which methane iodide and bromobenzene are preferred in order to obtain excellent heat resistance for resin films. These may be used alone or in combinations of two or more.

The process for producing a compound with an acetylene bond-containing group represented by general formula (5), of the compounds represented by general formula (1) may be, for example, a process wherein first a halogenated benzene is subjected to Friedel-Kraft reaction with a halogenated biadamantane compound in the presence of a Lewis acid such as aluminum bromide or aluminum chloride, to form a halogenated phenylbiadamantane compound, and then reacting the halogenated phenylbiadamantane compound with an ethynyl compound. Particularly in the case of a compound having hydrogen as R₆ in a group represented by general formula (5), a process may be mentioned wherein trimethylsilylacetylene is reacted as the ethynyl compound to form a trimethylsilylethynylphenylbiadamantane compound, and the trimethylsilylethynylphenylbiadamantane compound is detrimethylsilylated in the presence of an appropriate base (potassium carbonate or the like). In this case the compound represented by general formula (1) is a biadamantane compound, but the same description applies for other polyadamantane compounds wherein n is 2 or greater, as well as polyamantane compounds or poly(polyamantane) compounds, of the compounds represented by general formula (1).

The aforementioned example of a halogenated biadamantane compound employs the same starting materials as for synthesis of a biadamantane compound having an acetylene bond-containing group represented by general formula (4).

As examples of halogenated benzenes there may be mentioned bromobenzene, 1,2-dibromobenzene, 1,3-dibromobenzene, 1,4-dibromobenzene and 1,2,4-tribromobenzene, with no limitation to these.

In order to promote the reaction between the ethynyl compound and the halogenated phenylbiadamantane compound, an appropriate catalyst such as copper(II) iodide, triphenylphosphine or dichlorobis(triphenylphosphine)palladium may be added, and this may also be done for reaction between a halogenated alkane and acetylene group in the production process for a compound having an acetylene bond-containing group with an organic group as R₆, in the group represented by general formula (4) above.

As examples of ethynyl compounds there may be mentioned ethynyladamantane, ethynylbenzene, ethynylnaphthalene, 1-phenoxy-4-ethynylbenzene, ethynylfluorene, 4-(methylethynyl)ethynylbenzene and 4-(phenylethynyl)ethynylbenzene, which may be used alone or in combinations of two or more.

For synthesis of a compound represented by general formula (1) having hydrogen as R₆ in a group represented by general formula (5), the same process may be carried out as for reaction using trimethylsilylacetylene as an ethynyl compound.

As a process for producing a compound having a vinyl bond-containing group represented by general formula (6) of the compounds represented by general formula (1), there may be mentioned a process in which the halogenated hydrogen of a halogenated alkoxybiadamantane compound or a halogenated alkoxyphenylbiadamantane compound is removed in the presence of a base. In this case the compound represented by general formula (1) is a biadamantane compound, but the same description applies for other polyadamantane compounds wherein n is 2 or greater, as well as polyamantane compounds or poly(polyamantane) compounds, of the compounds represented by general formula (1).

As specific examples of such halogenated alkoxybiadamantane compounds there may be mentioned those with 3,3',5,5'-tetramethyl-1,1'-biadamantane structures, such as 3,3',5,5'-tetramethyl-7,7'-(3-bromopropoxy)-1,1'-biadamantane, 3,3',5,5'-tetramethyl-7,7'-(3-bromobutoxy)-1,1'-biadamantane, 3,3',5,5'-tetramethyl-7,7'-(4-bromobutoxy)-1,1'-biadamantane, 3,3',5,5'-tetramethyl-7,7'-(3-bromohexoxy)-1,1'-biadamantane, 3,3',5,5'-tetramethyl-7,7'-(4-bromohexoxy)-1,1'-biadamantane, 3,3',5,5'-tetramethyl-7,7'-(3-bromoheptoxy)-1,1'-biadamantane, 3,3',5,5'-tetramethyl-7,7'-(4-bromoheptoxy)-1,1'-biadamantane, 3,3',5,5'-tetramethyl-7,7'-(1,1-dimethyl-3-bromopropoxy)-1,1'-biadamantane, 3,3',5,5'-tetramethyl-7,7'-(1,1-diphenyl-3-bromopropoxy)-1,1'-biadamantane, 3,3',5,5'-tetramethyl-7,7'-(2,3-dimethyl-3-bromobutoxy)-1,1'-biadamantane and 3,3',5,5'-tetramethyl-7,7'-(2,3-diphenyl-3-bromobutoxy)-1,1'-biadamantane, with no limitation to these. The hydrogens in the aforementioned biadamantane compounds may be replaced by fluorine atoms, methyl, trifluoromethyl, phenyl or the like.

As specific examples of the aforementioned halogenated alkoxyphenylbiadamantane compounds there may be mentioned those with 3,3',5,5'-tetramethyl-1,1'-biadamantane structures, such as 3,3',5,5'-tetramethyl-7,7'-4-(3-bromopropoxy)phenyl-1,1'-biadamantane, 3,3',5,5'-tetramethyl-7,7'-4-(3-bromobutoxy)phenyl-1,1'-biadamantane, 3,3',5,5'-tetramethyl-7,7'-4-(4-bromobutoxy)phenyl-1,1'-biadamantane, 3,3',5,5'-tetramethyl-7,7'-4-(3-bromohexoxy)phenyl-1,1'-biadamantane, 3,3',5,5'-tetramethyl-7,7'-4-(4-bromohexoxy)phenyl-1,1'-biadamantane, 3,3',5,5'-tetramethyl-7,7'-4-(3-bromoheptoxy)phenyl-1,1'-biadamantane, 3,3',5,5'-tetramethyl-7,7'-4-(4-bromoheptoxy)phenyl-1,1'-biadamantane, 3,3',5,5'-tetramethyl-7,7'-4-(1,1-dimethyl-3-bromopropoxy)phenyl-1,1'-biadamantane, 3,3',5,5'-tetramethyl-7,7'-9-(1,1-diphenyl-3-bromopropoxy)phenyl-1,1'-biadamantane, 3,3',5,5'-tetramethyl-7,7'-4-(2,3-dimethyl-3-bromobutoxy)phenyl-1,1'-biadamantane and 3,3',5,5'-tetramethyl-7,7'-4-(2,3-diphenyl-3-bromobutoxy)phenyl-1,1'-biadamantane, with no limitation to these. The hydrogens in the aforementioned biadamantane compounds may be replaced by fluorine atoms, methyl, trifluoromethyl, phenyl or the like.

As a process for producing a compound having a vinyl bond-containing group with hydrogen as R₇ and R₈, in a group represented by general formula (6) among the compounds represented by general formula (1), there may be mentioned, for example, a synthesis process in which an appropriate reducing agent such as hydrogen or hydrazine is reacted with a compound represented by general formula (1) having an acetylene bond-containing group represented by general formula (3).

As a process for producing a compound having a vinyl bond-containing group represented by general formula (7) of the compounds represented by general formula (1), there may be mentioned a process in which the halogenated hydrogen of a halogenated alkyladamantane compound is removed in the presence of a base. In this case the compound represented by general formula (1) is a biadamantane compound, but the same description applies for other polyadamantane compounds wherein n is 2 or greater, as well as polyamantane compounds or poly(polyamantane) compounds, of the compounds represented by general formula (1).

As specific examples of the halogenated alkylbiadamantane compounds there may be mentioned those with 3,3',5,5'-tetramethyl-1,1'-biadamantane structures, such as 3,3',5,5'-tetramethyl-7,7'-(2-bromoethyl)-1,1'-biadamantane, 3,3',5,5'-tetramethyl-7,7'-(2-bromo-1-methylethyl)-1,1'-biadamantane, 3,3',5,5'-tetramethyl-7,7'-(2-bromo-1-phenylethyl)-1,1'-biadamantane, 3,3',5,5'-tetramethyl-7,7'-(2-bromo-2-phenylethyl)-1,1'-biadamantane, 3,3',5,5'-tetramethyl-7,7'-(2-bromo-1-methyl-2-phenylethyl)-1,1'-biadamantane, 3,3',5,5'-tetramethyl-7,7'-(2-bromopropyl)-1,1'-biadamantane, 3,3',5,5'-tetramethyl-7,7'-(2-bromo-1-methylpropyl)-1,1'-biadamantane, 3,3',5,5'-tetramethyl-7,7'-(2-bromo-1,2-dimethylpropyl)-1,1'-biadamantane, 3,3',5,5'-tetramethyl-7,7'-(2-bromo-1-methyl-2-phenylpropyl)-1,1'-biadamantane and 3,3',5,5'-tetramethyl-7,7'-(2-bromo-1,2-diphenylpropyl)-1,1'-biadamantane, with no limitation to these. The hydrogens in the aforementioned adamantane compounds may be replaced by fluorine atoms, methyl, trifluoromethyl, phenyl or the like.

As a process for producing a compound having a vinyl bond-containing group with hydrogen as R₇ and R₈, in a group of general formula (7) among the compounds represented by general formula (1), there may be mentioned, for example, a synthesis process in which an appropriate reducing agent such as hydrogen or hydrazine is reacted with a compound represented by general formula (1) having an acetylene bond-containing group represented by general formula (4).

As a process for producing a compound having a vinyl bond-containing group represented by general formula (8) of the compounds represented by general formula (1), there may be mentioned a process in which the halogenated hydrogen of a halogenated alkylphenylbiadamantane compound is removed in the presence of a base. In this case the compound represented by general formula (1) is a biadamantane compound, but the same description applies for other polyadamantane compounds wherein n is 2 or greater, as well as polyamantane compounds or poly(polyamantane) compounds, of the compounds represented by general formula (1).

As specific examples of the halogenated alkylphenylbiadamantane compounds there may be mentioned those with 3,3',5,5'-tetramethyl-1,1'-biadamantane structures, such as 3,3',5,5'-tetramethyl-7,7'-(4-(2-bromoethyl)phenyl)-1,1'-biadamantane, 3,3',5,5'-tetramethyl-7,7'-(3,5-di(2-bromoethyl)phenyl)-1,1'-biadamantane, 3,3',5,5'-tetramethyl-7,7'-(2,4,5-tri(2-bromoethyl)phenyl)-1,1'-biadamantane, 3,3',5,5'-tetramethyl-7,7'-(4-(2-bromo-2-phenylethyl)phenyl)-1,1'-biadamantane, 3,3',5,5'-tetramethyl-7,7'-(3,5-di(2-bromo-2-phenylethyl)phenyl)-1,1'-biadamantane, 3,3',5,5'-tetramethyl-7,7'-(2,4,5-tri(2-bromo-2-phenylethyl)phenyl)-1,1'-biadamantane, 3,3',5,5'-tetramethyl-7,7'-(4-(2-bromopropyl)phenyl)-1,1'-biadamantane, 3,3',5,5'-tetramethyl-7,7'-(3,5-di(2-bromopropyl)phenyl)-1,1'-biadamantane and 3,3',5,5'-tetramethyl-7,7'-(2,4,5-tri(2-bromopropyl)phenyl)-1,1'-biadamantane, with no limitation to these. The hydrogens in the aforementioned biadamantane compounds may be replaced by fluorine atoms, methyl, trifluoromethyl, phenyl or the like.

As a process for producing a compound having a vinyl bond-containing group with hydrogen as R₇ and R₈ in a group represented by general formula (8), among the compounds represented by general formula (1), there may be mentioned, for example, a synthesis process in which an appropriate reducing agent such as hydrogen or hydrazine is reacted with a compound represented by general formula (1) having an acetylene bond-containing group represented by general formula (5).

The polymer obtain by polymerizing a compound represented by general formula (1) in the organic insulating material of the invention may be obtained by reacting all or a portion of the polymerizable functional groups in the structure of the compound represented by general formula (1).

According to the invention, a polymer of a compound represented by general formula (1) may be an oligomer or a longer polymer, with a molecular weight of preferably between 1,000 and 300,000, and for fabrication of an organic insulating film it is preferably between 1,000 and 100,000 to exhibit satisfactory solubility in organic solvents, when it is to be used as a varnish.

The process for synthesizing a compound represented by general formula (1), for a polymer obtained by polymerization of a compound represented by general formula (1) to be used for the invention, may be any known polymerization process that permits reaction of the polymerizable functional groups, and as examples there may be mentioned a process by radical polymerization using a radical initiator such as benzoyl peroxide, t-butyl peroxide or azobisisobutyronitrile, a process by photoradical polymerization using photoirradiation, a process by polymerization or by thermal polymerization using a palladium catalyst such as dichlorobis(triphenylphosphine)palladium(II), bis(benzonitrile)palladium(II) dichloride or tetrakis(triphenylphosphine)palladium(0), a process by polymerization using a transition catalyst such as copper(II) acetate, a process by polymerization using a transition metal chloride such as molybdenum(V) chloride, tungsten(VI) chloride, tantalum(V) chloride or a Ziegler-Natta catalyst, or a process by ion polymerization using an anionic polymerization initiator such as n-butyllithium or s-butyllithium or a cationic polymerization initiator such as sulfuric acid, trifluoromethanesulfonic acid or trichloroacetic acid.

These polymerization processes proceed by reaction of all or a portion of the polymerizable functional groups in compounds represented by general formula (1). Examples of compounds represented by general formula (1) having acetylene bond-containing or vinyl bond-containing groups as groups with polymerizable functional groups will now be explained using simplified chemical formulas. When the compound represented by general formula (1) is represented by general formula (10) or (11), the structure of the compound represented by general formula (1) obtained by polymerization reaction may be one having a repeating unit represented by formula (12), for example, although there is no limitation to this structure. The polymerization mixture obtained by polymerization reaction may also include unreacted compounds represented by general formula (1).

≡-A-≡ (10)

(In formula (10), A is a structure other than a group containing a polymerizable functional group, in the structure represented by formula (1). Here, the group containing a polymerizable functional group is a group represented by general formula (4) above (where R₆ in general formula (4) is hydrogen), but another acetylene bond group may also be coordinated therewith.)

=-A-= (11)

(In formula (11), A is a structure other than a group containing a polymerizable functional group, in the structure represented by formula (1). Here, the group containing a polymerizable functional group is a group represented by general formula (7) above (where R₆, R₇ and R₈ in general formula (7) are hydrogen), but another vinyl bond group may also be coordinated therewith.)

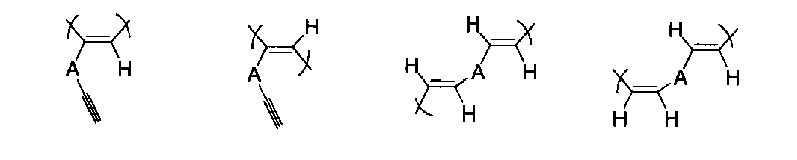

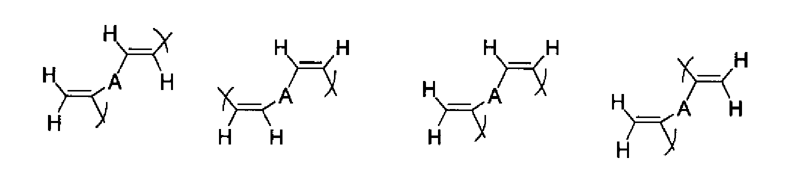

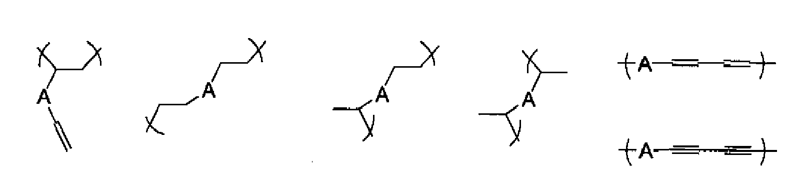

The examples of repeating units represented by formula (12) above include an example of reacting 1 or 2 acetylene bond groups in a compound represented by general formula (1) and an example of reacting 1 or 2 vinyl bond groups in a compound represented by general formula (1), but even more acetylene bond groups or vinyl bond groups may be reacted in a compound represented by general formula (1).

An organic solvent may be used as the reaction solvent for the aforementioned polymerization reactions, but there is no particular restriction to such organic solvents, and for example, alcohol-based solvents such as methanol, ethanol, isopropanol, 1-butanol and 2-butanol; ketone-based solvents such as acetylacetone, methyl ethyl ketone, methyl isobutyl ketone, cyclohexanone, cyclopentanone, 2-pentanone and 2-heptanone; esteric solvents such as ethyl acetate, propyl acetate, butyl acetate, pentyl acetate and propyleneglycol monomethyl ether acetate; ether-based solvents such as diisopropyl ether, dibutyl ether, tetrahydrofuran, anisole and 1,3-dimethoxybenzene; aromatic and aliphatic hydrocarbon-based solvents such as benzene, toluene, mesitylene, ethylbenzene, diethylbenzene, propylbenzene, heptane and hexane; and amide-based solvents such as N-methylpyrrolidone, are industrially available and therefore suitable solvents, and any of these may be used alone or in combinations of two or more.

An organic insulating material according to the invention is obtained from a compound represented by general formula (1), or a polymer obtained by polymerizing a compound represented by general formula (1), or a mixture of a compound represented by general formula (1) and the aforementioned polymer, and in order to obtain uniform resin films using the material, it preferably contains a polymer obtained by polymerizing a compound represented by general formula (1), or a mixture of a compound represented by general formula (1) and said polymer. In the case of a mixture of a compound represented by general formula (1) and the aforementioned polymer, the proportion of the compound represented by general formula (1) with respect to the total weight of the compound represented by general formula (1) and said polymer is preferably between 1 wt% and 60 wt%, and more preferably between 1 wt% and 30 wt%. The organic insulating material of the invention may also contain, in addition to these components, other additives that can optionally be used in organic insulating film varnishes.

A varnish for organic insulating film according to the invention can be obtained by dissolving the aforementioned organic insulating material in a suitable organic solvent. The organic insulating material may be dried and hardened and then dissolved in an organic solvent to produce a varnish for organic insulating film, or the reaction mixture obtained by production of the organic insulating material may be directly used as a varnish. As organic solvents to be used for resin film varnishes there may be mentioned any ones that can dissolve or disperse the organic insulating material, without any particular restrictions, and the same organic solvent used for the polymerization reaction may be employed. The concentration of the organic insulating film varnish may be set as appropriate for the structure and molecular weight of the organic insulating material, but the organic insulating material is preferably used at between 0.1 wt% and 50 wt% and more preferably between 0.5 wt% and 15 wt% in the varnish for organic insulating film.

If necessary, there may be added to the varnish for organic insulating film various additives including surfactants, coupling agents of which silane coupling agents are typical, radical initiators that generate oxygen radicals or sulfur radicals under heat, and catalysts such as disulfides.

Addition of a naphthoquinonediazide compound as a photosensitive agent to the varnish for organic insulating film will allow its use as a surface protecting film with a photosensitive property.

A foaming agent that forms nanosize micropores (porogen, or pore generator) in insulating films may also be added to the varnish for organic insulating film.

As examples of foaming agents there may be mentioned carbon nanotubes or fullerene with a hollow structure, cage silsesquioxane, cyclodextrin, high-melting-point organic compounds, surfactants, azobis compounds, organic peroxides, dendrimers comprising polyamideamine structures, polymethacrylic acid-based hyperbranch polymers, and the like. Surfactants and hyperbranch polymers are preferred among these. This will allow the foaming agent to be uniformly dispersed in the organic insulating material. If the foaming agent is uniformly dispersed, further heating and extraction treatment can yield an organic insulating film with homogeneous micropores.

The organic insulating film will now be explained.

The organic insulating film of the invention is obtained using the aforementioned organic insulating material or varnish for organic insulating film. For example, it may be produced by coating a varnish for organic insulating film obtained as described above onto a support such as a panel and treating it by heat or an active energy beam. It may also be produced by coating a support with the polymerization mixture obtained as described above directly or with the organic insulating material after heating and dissolution. Treatment by heating or an active energy beam can accomplish crosslinking reaction of the polymerizable functional groups of the compound represented by general formula (1), or the polymerizable functional groups remaining without reacting when the polymer is obtained, or the functional groups remaining after the polymerizable functional groups have reacted when the polymer is obtained, by the heating or active energy beam treatment, to provide an organic insulating film with an excellent elastic modulus.

A concrete example of using the aforementioned varnish for organic insulating film in an organic insulating film production process of the invention will now be explained. First, the varnish for organic insulating film is coated onto a suitable support, for example an organic base such as a polyester film, a metal sheet such as a copper foil, or a semiconductor board such as a silicon wafer or ceramic board, to form a coated film. The coating method may be spin coating using a spinner, spray coating using a spray coater, dipping, printing, roll coating or the like. The coated film may then be treated with heat or the like for drying, the solvent in the coated film removed, and then the dried coated film subjected to crosslinking reaction by a method of heating, a method of irradiating an active energy beam or a method involving both, to obtain an organic insulating film with excellent mechanical properties. The method by heating may involve, for example, heating at 150-425°C x 5 minutes-24 hours. As active energy beams there may be mentioned active energy light rays such as visible light, ultraviolet rays, infrared light or laser light, or X-rays, electron beams and microwaves.

The organic insulating film of the invention may be directly coated onto a substrate such as a semiconductor board by a method mentioned above, or a resin film formed on a support such as an organic base material may be released from the support for use as a dry film.

As examples of uses for the organic insulating film, there may be mentioned semiconductor interlayer insulating films and surface protecting films, multilayer circuit interlayer insulating films, flexible copper-clad sheet cover coats, solder resist films, liquid crystal oriented films, etching protective films (etching stoppers), adhesives and the like. Most suitable among these are semiconductor interlayer insulating films, surface protecting films and etching protective films.

The glass transition temperature of the organic insulating material used is not particularly restricted but is preferably at least 350°C and most preferably at least 400°C. With a glass transition temperature within this range, the linear expansion coefficient of the organic insulating film can be reduced and an organic insulating film with excellent dimensional stability can be obtained.

The thickness of the organic insulating film is not particularly restricted, but it is preferably 0.01-20 µm, more preferably 0.05-10 µm and most preferably 0.1-0.7 µm, for a semiconductor interlayer insulating film or the like. A thickness within this range will result in excellent suitability for semiconductor production processes.

When the organic insulating film is used as a semiconductor interlayer insulating film, the organic insulating material or varnish for organic insulating film is directly coated onto prescribed locations of a silicon wafer or ceramic board to form a coated film. The coating method may be spin coating using a spinner, spray coating using a spray coater, dipping, printing, roll coating or the like. The coated film may then be dried, the solvent in the coated film removed, and then the dried coated film subjected to crosslinking reaction by a method of heating as mentioned above, a method of irradiating an active energy beam, or a method involving both, to obtain an interlayer insulating film. Alternatively, the varnish for organic insulating film may be used as a dry film and laminated at the prescribed location.

When the organic insulating film is used as a semiconductor protecting film, the organic insulating material or varnish for organic insulating film is directly coated onto prescribed locations of a silicon wafer or ceramic board to form a coated film, in the same manner as a semiconductor interlayer insulating film explained above. The coating method may be spin coating using a spinner, spray coating using a spray coater, dipping, printing, roll coating or the like. The coated film may then be dried, the solvent in the coated film removed, and then the dried coated film subjected to crosslinking reaction by a method of heating as mentioned above, a method of irradiating an active energy beam, or a method involving both, to obtain a protecting film composed of the organic insulating film.

The thickness of the semiconductor protecting film is not particularly restricted, but it is preferably 0.01-70 µm and more preferably 0.05-50 µm. A thickness within this range will result in excellent protective properties and workability of the semiconductor element.

A preferred embodiment of a semiconductor device will now be described, employing an organic insulating film according to the invention as the interlayer insulating film.

Fig. 1 is a schematic cross-sectional view showing an example of a semiconductor device according to the invention.

The semiconductor device 100 comprises a semiconductor board 1 on which an element is formed, a silicon nitride film 2 provided above the semiconductor board 1 (the top in Fig. 1), and a copper wiring layer 7 covered with an interlayer insulating film 3 and a barrier layer 6, formed on the silicon nitride film 2.

Recesses corresponding to a wiring pattern are formed in the interlayer insulating film 3, and the copper wiring layer 7 is formed in the recesses.

A reforming treatment layer 5 is provided between the interlayer insulating film 3 and copper wiring layer 7.

A hard mask layer 4 is also formed above the interlayer insulating film 3 (the side opposite the silicon nitride film 2).

As the method for fabricating the semiconductor device 100, first a semiconductor board 1 comprising a device such as a transistor on a silicon wafer is prepared, and a silicon nitride film 2 is formed thereover as an insulating layer, while the interlayer insulating film 3 and hard mask layer 4 are further formed thereover. A photoresist layer is additionally formed thereover, and wiring grooves are formed by dry etching, through prescribed sections of the insulating layer comprising the interlayer insulating film and hard mask layer. Next, the reforming treatment layer 5 is formed by plasma treatment inside the wiring grooves, and then the barrier layer 6 composed of Ta, Ti, TaN, TiN, WN or the like is formed by PVD or CVD. A copper layer 7 serving as the wiring layer is then formed by electric field plating or the like, after which the sections of the copper layer and barrier metal layer other than the wiring sections are removed by polishing and flattened, using CMP, to produce a semiconductor device 100.

As a specific method for forming the interlayer insulating film 3, the varnish for organic insulating film may be directly coated onto the silicon nitride film 2 of the semiconductor board 1 to form the interlayer insulating film, but alternatively it may be formed by preparing a dry film of the organic insulating film beforehand and laminating it on the silicon nitride film 2 of the semiconductor board 1. More specifically, a varnish for organic insulating film obtained in the manner described above may be directly coated onto the silicon nitride film 2 of the semiconductor board 1 to form a coated film, and the coated film dried and then irradiated by heat and/or an active energy beam for hardening to form an interlayer insulating film 3. When a dry film is used, the varnish for organic insulating film obtained in the manner described above may be used to form a resin layer on a substrate and dried to form a dry film, which is then laminated on the silicon nitride film 2 of the semiconductor board 1 and irradiated with heat and/or an active energy beam to harden the dry film and form an interlayer insulating film 3.

The explanation provided above assumes an example of formation on the silicon nitride film 2, but the position of the organic insulating film is not limited to this.

A semiconductor device 100 employing an interlayer insulating film 3 was explained for this embodiment, but the invention is not limited to such a construction.

Since an interlayer insulating film as described above is used in the semiconductor device of the invention, the dimensional precision is excellent and a sufficient insulating property is exhibited, so that excellent connection reliability is obtained.

Moreover, since the interlayer insulating film as described above has an excellent elastic modulus, it is suitable for processes for forming wirings on semiconductor devices.

Furthermore, since the interlayer insulating film as described above also has excellent dielectric characteristics, the wiring delay can be reduced.

### Examples

The present invention will now be explained in greater detail based on examples and comparative examples, with the understanding that the invention is in no way limited to the examples.

### (Example 1)

### (1) [Synthesis of 3,3',5,5'-tetramethyl-7,7'-bis(2-propynyloxy)-1,1'-biadamantane]

After loading 3.6 g (10 mmol) of 3,3'-dihydroxy-5,5',7,7'-tetramethyl-1,1'-biadamantane, 23 g (193 mmol) of 3-bromo-1-propyne, 2 g (50 mmol) of sodium hydroxide, 60 mL of tetrahydrofuran, 60 mL of water and a stirrer in a 300 mL volumetric flask, the mixture was stirred at 65°C for 5 hours under a nitrogen stream to obtain a reaction mixture. The organic liquid layer of the reaction mixture was recovered, the solvent was removed, and then 200 mL of methanol was added and stirred therewith, after which the precipitated crystals were filtered and dried under reduced pressure to obtain 2.4 g of 3,3',5,5'-tetramethyl-7,7'-bis(2-propynyloxy)-1,1'-biadamantane.

The appearance of the obtained compound was visually examined. The compound was cationized (M⁺) by field desorption (FD), and the mass (m/z) of the compound was measured by mass spectrometry (MS) while each of the elements in the compound were also quantified by elemental analysis. The compound was identified based on the following measurement results. Outer appearance: white solid
MS (FD)(m/z): 434 (M⁺)
Elemental analysis: Calculated (/%): C; 82.90, H; 9.74, O; 7.36, Found (/%): C; 82.85, H; 9.76, O; 7.39

### (2) Polymerization of polyadamantane compound and production of varnish for organic insulating film

After dissolving 5 g of a compound obtained by the same procedure as Example 1(1) in 45 g of 1,3-dimethoxybenzene, reaction was conducted at 190°C for 6 hours under dry nitrogen, and the reaction mixture was dropped into a 10-fold volume of methanol to produce a precipitate, which was collected and dried to obtain a polymer. The molecular weight of the obtained polymer was determined using a gel permeation chromatograph (GPC) by Tosoh Corp. based on polystyrene, and the weight-average molecular weight was 62,200. A 3 g portion of the obtained polymer was dissolved in 27 g of cyclopentanone and filtered with a Teflon^{R} filter to obtain a varnish for organic insulating film.

### (3) Production of interlayer insulating film and semiconductor device

A silicon nitride layer was formed on a semiconductor board, and the aforementioned varnish for organic insulating film was coated onto the silicon nitride layer and subjected to heat treatment at 400°C for 1 hour to form an interlayer insulating film with a 0.1 µm thickness.

Next, a metal wiring was formed on the interlayer insulating film in a prescribed pattern, to obtain a semiconductor device.

### (Example 2)

### (1) [Synthesis of 3,3',5,5'-tetrakis(2-propynyloxy)-1,1'-biadamantane]

After loading 3.3 g (10 mmol) of 3,3',5,5'-tetrahydroxy-1,1'-biadamantane, 23 g (193 mmol) of 3-bromo-1-propyne, 3 g (75 mmol) of sodium hydroxide, 60 mL of tetrahydrofuran, 60 mL of water and a stirrer in a 300 mL volumetric flask, the mixture was stirred at 65°C for 5 hours under a nitrogen stream to obtain a reaction mixture. The organic liquid layer of the reaction mixture was recovered, the solvent was removed, and then 200 mL of methanol was added and stirred therewith, after which the precipitated crystals were filtered and dried under reduced pressure to obtain 2.3 g of 3,3',5,5'-tetrakis(2-propynyloxy)-1,1'-biadamantane.

The previously obtained compound was used for evaluation in the same manner as Example 1, whereby it was confirmed to be the same compound. The measurement results are shown below. Outer appearance: white solid
MS (FD)(m/z): 486 (M⁺)
Elemental analysis: Calculated (/%): C; 78.98, H; 7.87,
O; 13.15, Found (/%): C; 78.92, H; 7.79, O; 13.29

### (2) Polymerization of polyadamantane compound and production of varnish for organic insulating film

After dissolving 3 g of a compound obtained by the same procedure as Example 2(1) in 27 g of toluene, 0.3 g of tungsten(VI) chloride was added, reaction was conducted at 30°C for 24 hours under dry nitrogen, and the reaction mixture was dropped into a 10-fold volume of methanol to produce a precipitate, which was collected and dried to obtain a polymer. The weight-average molecular weight of the obtained polymer was 92,600. A 2 g portion of the obtained polymer was dissolved in 18 g of cyclopentanone and filtered with a filter to obtain a varnish for organic insulating film.

### (3) Production of interlayer insulating film and semiconductor device

The previously obtained varnish for organic insulating film was used for the same procedure as Example 1(3) to obtain an interlayer insulating film and semiconductor device.

### (Example 3)

### (1) [Synthesis of 3,3',5,5'-tetramethyl-7,7'-bis(4-(2-propynyloxy)phenyl)-1,1'-biadamantane]

After loading 3.2 g (6 mmol) of 3,3'-bis(4-hydroxyphenyl)-5,5',7,7'-tetramethyl-1,1'-biadamantane, 17 g (143 mmol) of 3-bromo-1-propyne, 2 g (50 mmol) of sodium hydroxide, 60 mL of tetrahydrofuran, 60 mL of water and a stirrer in a 300 mL volumetric flask, the mixture was stirred at 65°C for 5 hours under a nitrogen stream to obtain a reaction mixture. The organic liquid layer of the reaction mixture was recovered, the solvent was removed, and then 200 mL of methanol was added and stirred therewith, after which the precipitated crystals were filtered and dried under reduced pressure to obtain 2.7 g of 3,3',5,5'-tetramethyl-7,7'-bis(4-(2-propynyloxy)phenyl)-1,1'-biadamantane.

The previously obtained compound was used for evaluation in the same manner as Example 1, whereby it was confirmed to be the same compound. The measurement results are shown below. Outer appearance: white solid
MS (FD)(m/z): 586 (M⁺)
Elemental analysis: Calculated (/%): C; 85.96, H; 8.59, O; 5.45, Found (/%): C; 86.02, H; 8.55, O; 5.43

### (2) Polymerization of polyadamantane compound and production of varnish for organic insulating film

After dissolving 5 g of a compound obtained by the same procedure as Example 3(1) in 95 g of toluene, 0.5 g of tantalum(V) chloride was added, reaction was conducted at 30°C for 24 hours under dry nitrogen, and the reaction mixture was dropped into a 10-fold volume of methanol to produce a precipitate, which was collected and dried to obtain a polymer. The weight-average molecular weight of the obtained polymer was 85,100. A 3 g portion of the obtained polymer was dissolved in 27 g of cyclopentanone and filtered with a filter to obtain a varnish for organic insulating film.

### (3) Production of interlayer insulating film and semiconductor device

The previously obtained varnish for organic insulating film was used for the same procedure as Example 1(3) to obtain an interlayer insulating film and semiconductor device.

### (Example 4)

### (1) [Synthesis of 3,3',5,5'-tetrakis(4-(2-propynyloxy)phenyl)-2,2'-biadamantane]

After loading 3.2 g (5 mmol) of 3,3',5,5'-tetrakis(4-hydroxyphenyl)-2,2'-biadamantane, 22 g (185 mmol) of 3-bromo-1-propyne, 3.2 g (80 mmol) of sodium hydroxide, 60 mL of tetrahydrofuran, 60 mL of water and a stirrer in a 300 mL volumetric flask, the mixture was stirred at 65°C for 6 hours under a nitrogen stream to obtain a reaction mixture. The organic liquid layer of the reaction mixture was recovered, the solvent was removed, and then 200 mL of methanol was added and stirred therewith, after which the precipitated crystals were filtered and dried under reduced pressure to obtain 3.6 g of 3,3',5,5'-tetrakis(4-(2-propynyloxy)phenyl)-2,2'-biadamantane.

The previously obtained compound was used for evaluation in the same manner as Example 1, whereby it was confirmed to be the same compound. The measurement results are shown below. Outer appearance: white solid
MS (FD)(m/z): 790 (M⁺)
Elemental analysis: Calculated (/%): C; 85.03, H; 6.88,
O; 8.09, Found (/%): C; 85.10, H; 6.87, O; 8.03

### (2) Polymerization of polyadamantane compound and production of varnish for organic insulating film

After dissolving 5 g of a compound obtained by the same procedure as Example 4(1) in 45 g of 1,3-dimethoxybenzene, 0.1 g of bis(benzonitrile)palladium(II) dichloride was added, reaction was conducted at 190°C for 6 hours under dry nitrogen, and the reaction mixture was dropped into a 10-fold volume of methanol to produce a precipitate, which was collected and dried to obtain a polymer. The weight-average molecular weight of the obtained polymer was 19,600. A 3 g portion of the obtained polymer was dissolved in 27 g of cyclopentanone and filtered with a filter to obtain a varnish for organic insulating film.

### (3) Production of interlayer insulating film and semiconductor device

The previously obtained varnish for organic insulating film was used for the same procedure as Example 1(3) to obtain an interlayer insulating film and semiconductor device.

### (Example 5)

### (1) [Synthesis of 3,3',5,5'-tetramethyl-7,7'-bis(2,4-bis(2-propynyloxy)phenyl)-1,1'-biadamantane]

After loading 3.1 g (6 mmol) of 3,3'-bis(2,4-dihydroxyphenyl)-5,5',7,7'-tetramethyl-1,1'-biadamantane, 25 g (210 mmol) of 3-bromo-1-propyne, 4 g (100 mmol) of sodium hydroxide, 60 mL of tetrahydrofuran, 60 mL of water and a stirrer in a 300 mL volumetric flask, the mixture was stirred at 65°C for 5 hours under a nitrogen stream to obtain a reaction mixture. The organic liquid layer of the reaction mixture was recovered, the solvent was removed, and then 200 mL of methanol was added and stirred therewith, after which the precipitated crystals were filtered and dried under reduced pressure to obtain 2.9 g of 3,3',5,5'-tetramethyl-7,7'-bis(2,4-bis(2-propynyloxy)phenyl)-1,1'-biadamantane.

The previously obtained compound was used for evaluation in the same manner as Example 1, whereby it was confirmed to be the same compound. The measurement results are shown below. Outer appearance: white solid
MS (FD)(m/z): 694 (M⁺)
Elemental analysis: Calculated (/%): C; 82.96, H; 7.83, O; 9.21, Found (/%): C; 83.01, H; 7.83, O; 9.16

### (2) Polymerization of polyadamantane compound and production of varnish for organic insulating film

After dissolving 5 g of a compound obtained by the same procedure as Example 5(1) in 45 g of 1,3-dimethoxybenzene, 0.1 g of bis(triphenylphosphonium)palladium(II) dichloride was added, reaction was conducted at 190°C for 6 hours under dry nitrogen, and the reaction mixture was dropped into a 10-fold volume of methanol to produce a precipitate, which was collected and dried to obtain a polymer. The weight-average molecular weight of the obtained polymer was 47,500. A 3 g portion of the obtained polymer was dissolved in 27 g of cyclopentanone and filtered with a filter to obtain a varnish for organic insulating film.

### (3) Production of interlayer insulating film and semiconductor device

The previously obtained varnish for organic insulating film was used for the same procedure as Example 1(3) to obtain an interlayer insulating film and semiconductor device.

### (Example 6)

### (1) [Synthesis of 3,3',5,5'-tetraethynyl-1,1'-biadamantane]

After placing 14 g (0.6 mol) of metallic sodium and 600 ml of n-octane in a 1000 mL four-necked flask equipped with a thermometer, stirrer and reflux tube, the internal temperature was cooled to 0°C. Next, 64.5 g (0.3 mol) of 1-bromoadamantane pre-dissolved in 300 ml of n-octane was slowly added dropwise while vigorously stirring. The internal temperature during the dropwise addition was kept at 0°C-5°C. Upon cessation of temperature increase after completion of the dropwise addition, reaction was continued for 1 hour. The mixture was then poured into approximately 1500 mL of cold water and the crude product was filtered out, washed with purified water and dried. The crude product was recrystallized from hot hexane. The obtained recrystallized product was dried under reduced pressure to obtain 32.6 g of a product. The Br group absorption (near 690-515 cm⁻¹) in IR analysis disappeared and mass spectrometry revealed a molecular weight of 270, thus indicating that the product was 1,1'-biadamantane.

After placing 700 mL of carbon tetrachloride and 70 g (0.44 mol) of bromine in a 2000 mL 4-necked flask equipped with a thermometer, stirrer and reflux tube, 54.1 g (0.2 mol) of the previously obtained 1,1'-biadamantane was added in small portions at a time while stirring. The internal temperature was kept at 20°C-30°C during the addition. Upon cessation of temperature increase after completion of the addition, reaction was continued for 1 hour. The mixture was then poured into approximately 2000 mL of cold water and the crude product was filtered out, washed with purified water and dried. The crude product was recrystallized from hot ethanol. The obtained recrystallized product was dried under reduced pressure to obtain 65.0 g of a product. When the obtained product was examined by infrared spectroscopic analysis (IR analysis), bromo group absorption was seen at 690-515 cm⁻¹ and the molecular weight by mass spectrometry was 586, thus indicating that the product was 3,3',5,5'-tetrabromo-1,1'-biadamantane.

After dissolving 20 g (34 mmol) of the obtained 3,3',5,5'-tetrabromo-1,1'-biadamantane and 18 ml (256 mmol) of bromoethene in 120 ml of dichloromethane in a flask, 3.0 g (22 mmol) of aluminum(III) chloride was added dropwise at -15°C under dry nitrogen and the mixture was stirred for 1 hour. After then adding 20 ml of water dropwise at -15°C, it was returned to room temperature to obtain a reaction mixture. The obtained reaction mixture was added to 200 ml of a 10% hydrochloric acid aqueous solution, the mixture was extracted 3 times using 40 ml of dichloromethane each time and then washed with 40 ml of water and dried over magnesium sulfate, and the organic layer was concentrated to obtain 18.2 g of 3,3',5,5'-tetra(dibromoethyl)-1,1'-biadamantane.

The obtained 3,3',5,5'-tetra(dibromoethyl)-1,1'-biadamantane was dissolved in 200 ml of dimethyl sulfoxide, and then 28 g (250 mmol) of potassium tert-butoxide was added at room temperature and the mixture was stirred for 48 hours. The reaction mixture was added to 400 ml of water, the mixture was extracted 3 times using 200 ml of dichloromethane each time and then washed with 200 ml of water and dried over magnesium sulfate, and the organic layer was concentrated to obtain 11.0 g of 3,3',5,5'-tetraethynyl-1,1'-biadamantane.

The previously obtained compound was used for evaluation in the same manner as Example 1, whereby it was confirmed to be the same compound. The measurement results are shown below. Outer appearance: white solid
MS (FD)(m/z): 367 (M⁺)
Elemental analysis: Calculated (/%): C; 91.75, H; 8.25, Found (/%): C; 91.54, H; 8.13

### (2) Polymerization of polyadamantane compound and production of varnish for organic insulating film

A polymer was obtained in the same manner as Example 4(2), except that 5 g of the compound obtained in Example 6(1) was used instead of 5 g of a compound obtained by the same procedure as Example 4(1), used in Example 4(2). The weight-average molecular weight of the obtained polymer was 50,800. A 3 g portion of the obtained polymer was dissolved in 27 g of cyclopentanone and filtered with a filter to obtain a varnish for organic insulating film.

### (3) Production of interlayer insulating film and semiconductor device

The previously obtained varnish for organic insulating film was used for the same procedure as Example 1(3) to obtain an interlayer insulating film and semiconductor device.

### (Example 7)

### (1) [Synthesis of 3,3'-diethynyl-5,5',7,7'-tetramethyl-1,1'-biadamantane]

Except for using 72.9 g (0.3 mol) of 1-bromo-3,5-dimethyladamantane instead of 64.5 g (0.3 mol) of 1-bromoadamantane and using 35 g (0.22 mol) of bromine, as in Example 6(1), the procedure was otherwise carried out in the same manner as Example 6(1) to obtain 58 g of a product. Bromo group absorption was seen at 690-515 cm⁻¹ by IR analysis and the molecular weight by mass spectrometry was 484, thus indicating that the product was 3,3',5,5'-tetramethyl-7,7'-dibromo-1,1'-biadamantane.

Except for using 50 g (103 mmol) of the obtained 3,3',5,5'-tetramethyl-7,7'-dibromo-1,1'-biadamantane instead of 20 g (34 mmol) of 3,3',5,5'-tetrabromo-1,1'-biadamantane, 27.25 ml (387.5 mmol) instead of 18 ml (256 mol) of bromoethene and 4.55 g (33.3 mmol) instead of 3.0 g (22 mol) of aluminum(III) chloride in Example 6(1), the procedure was otherwise carried out as in Example 6(1) to obtain 31.5 g of 3,3'-diethynyl-5,5',7,7'-tetramethyl-1,1'-biadamantane.

The previously obtained compound was used for evaluation in the same manner as Example 1, whereby it was confirmed to be the same compound. The measurement results are shown below. Outer appearance: white solid
MS (FD)(m/z): 374 (M⁺)
Elemental analysis: Calculated (/%): C; 89.78, H; 10.22, Found (/%): C; 89.70, H; 10.13

### (2) Polymerization of polyadamantane compound and production of varnish for organic insulating film

A polymer was obtained in the same manner as Example 5(2), except that 5 g of the compound obtained in Example 7(1) was used instead of 5 g of a compound obtained by the same procedure as Example 5(1), used in Example 5(2). The weight-average molecular weight of the obtained polymer was 68,300. A 3 g portion of the obtained polymer was dissolved in 27 g of cyclopentanone and filtered with a filter to obtain a varnish for organic insulating film.

### (3) Production of interlayer insulating film and semiconductor device

The previously obtained varnish for organic insulating film was used for the same procedure as Example 1(3) to obtain an interlayer insulating film and semiconductor device.

### (Example 8)

### (1) [Synthesis of 4,9-diethynyldiamantane]

Except for using 30 g (87 mmol) of 4,9-dibromodiamantane instead of 20 g (34 mmol) of 3,3',5,5'-tetrabromo-1,1'-biadamantane, 23.4 ml (326 mmol) instead of 18 ml (256 mmol) of bromoethene and 3.7 g (28 mmol) instead of 3.0 g (22 mmol) of aluminum(III) chloride in Example 6(1), the procedure was otherwise carried out in the same manner as Example 6(1) to obtain 17 g of 4,9-diethynyldiamantane.

The previously obtained compound was used for evaluation in the same manner as Example 1, whereby it was confirmed to be the same compound. The measurement results are shown below. Outer appearance: white solid
MS (FD)(m/z): 236 (M⁺)
Elemental analysis: Calculated (/%): C; 91.47, H; 8.53, Found (/%): C; 91.38, H; 8.49

### (2) Polymerization of polyamantane compound and production of varnish for organic insulating film

A polymer was obtained in the same manner as Example 4(2), except that 5 g of the compound obtained in Example 8(1) was used instead of 5 g of a compound obtained by the same procedure as Example 4(1), used in Example 4(2). The weight-average molecular weight of the obtained polymer was 87,900. A 3 g portion of the obtained polymer was dissolved in 27 g of cyclopentanone and filtered with a filter to obtain a varnish for organic insulating film.

### (3) Production of interlayer insulating film and semiconductor device

The previously obtained varnish for organic insulating film was used for the same procedure as Example 1(3) to obtain an interlayer insulating film and semiconductor device.

### (Example 9)

### (1) [Synthesis of 3,3'-bis(phenylethynyl)-5,5',7,7'-tetramethyl-1,1'-biadamantane]

After dissolving 10 g (26.7 mmol) of the 3,3'-diethynyl-5,5',7,7'-tetramethyl-1,1'-biadamantane obtained in Example 7(1) and 12.5 g (79.6 mmol) of bromobenzene in 40 ml of triethylamine and 20 ml of pyridine in a flask, 0.062 g (0.33 mmol) of copper(II) iodide and 0.24 g (0.91 mmol) of triphenylphosphine were added. Next, 0.058 g (0.082 mmol) of dichlorobis(triphenylphosphine)palladium(II) was added and the mixture was reacted at 110°C for 5 hours under a dry nitrogen atmosphere. After the reaction, the triethylamine and pyridine were distilled off and 500 ml of a 2 mol/L hydrochloric acid aqueous solution was added to deposit a precipitate. The precipitate was filtered and washed with 500 ml of water and 500 ml of methanol, and then a vacuum dryer was used for 24 hours of drying in a 60°C atmosphere to obtain 9.7 g of 3,3'-bis(phenylethynyl)-5,5',7,7'-tetramethyl-1,1'-biadamantane.

The previously obtained compound was used for evaluation in the same manner as Example 1, whereby it was confirmed to be the same compound. The measurement results are shown below. Outer appearance: white solid
MS (FD)(m/z): 526 (M⁺)
Elemental analysis: Calculated (/%): C, 91.20; H, 8.80, Found (/%): C, 91.17; H, 8.79

### (2) Polymerization of polyadamantane compound and production of varnish for organic insulating film

A polymer was obtained in the same manner as Example 3(2), except that 5 g of the compound obtained in Example 9(1) was used instead of 5 g of a compound obtained by the same procedure as Example 3(1), used in Example 3(2). The weight-average molecular weight of the obtained polymer was 71,100. A 3 g portion of the obtained polymer was dissolved in 27 g of cyclopentanone and filtered with a filter to obtain a varnish for organic insulating film.

### (3) Production of interlayer insulating film and semiconductor device

The previously obtained varnish for organic insulating film was used for the same procedure as Example 1(3) to obtain an interlayer insulating film and semiconductor device.

### (Example 10)

### (1) [Synthesis of 3,3'''-dimethylethynyl-5,7,5',7',5'',7'',5''',7'''-octamethyl-1,1':3',1'':3'',1'''-tetraadamantane]

After dissolving 55 g (68 mmol) of 3,3'''-dibromo-5,7,5',7',5'',7'',5''',7'''-octamethyl-1,1':3',1'':3'',1'''-tetraadamantane and 18 ml (256 mmol) of bromoethene in 240 ml of dichloromethane in a flask, 3.0 g (22 mmol) of aluminum(III) chloride was added dropwise at -15°C under dry nitrogen and the mixture was stirred for 1 hour. After then adding 40 ml of water dropwise at -15°C, it was returned to room temperature to obtain a reaction mixture. The reaction mixture was added to 400 ml of a 10% hydrochloric acid aqueous solution and extracted 3 times using 80 ml of dichloromethane each time, and then washed with 80 ml of water and dried over magnesium sulfate, and the organic layer was concentrated to obtain 50.2 g of 3,3'''-dibromoethyl-5,7,5',7',5'',7',5''',7'''-octamethyl-1,1':3',1'':3'',1'''-tetraadamantane.

The obtained 3,3'''-dibromoethyl-5,7,5',7',5'',7'',5''',7'''-octamethyl-1,1':3',1'':3'',1'''-tetraadamantane was then dissolved in 400 ml of dimethyl sulfoxide and 28 g (250 mmol) of potassium tert-butoxide was added at room temperature, after which the mixture was stirred for 48 hours. The reaction mixture was then added to 800 ml of water and extracted 3 times using 400 ml of dichloromethane each time, and after washing with 400 ml of water, it was dried over magnesium sulfate and the organic layer was concentrated to obtain 36.1 g of 3,3'''-diethynyl-5,7,5',7',5'',7'',5''',7'''-octamethyl-1,1':3',1'':3'',1'''-tetraadamantane.

Next, 36.1 g of the obtained 3,3'''-diethynyl-5,7,5',7',5'',7'',5''',7'''-octamethyl-1,1':3',1'':3'',1'''-tetraadamantane and 22.7 g (160 mmol) of methyl iodide were dissolved in 80 ml of triethylamine and 40 ml of pyridine, and then 0.124 g (0.66 mmol)g of copper(II) iodide and 0.48 g (1.82 mmol) of triphenylphosphine were added. Next, 0.116 g (0.164 mmol) of dichlorobis(triphenylphosphine)palladium(II) was added and the mixture was reacted at 110°C for 5 hours under a dry nitrogen atmosphere. After the reaction, the triethylamine and pyridine were distilled off and 1000 ml of a 2 mol/L hydrochloric acid aqueous solution was added to deposit a precipitate. The precipitate was filtered and washed with 1000 ml of water and 1000 ml of methanol, and then a vacuum dryer was used for 24 hours of drying in a 60°C atmosphere to obtain 32.8 g of 3,3'''-dimethylethynyl-5,7,5',7',5'',7'',5''',7'''-octamethyl-1,1':3',1'':3'',1'''-tetraadamantane.

The previously obtained compound was used for evaluation in the same manner as Example 1, whereby it was confirmed to be the same compound. The measurement results are shown below. Outer appearance: white solid
MS (FD)(m/z): 727 (M⁺)
Elemental analysis: Calculated (/%): C; 89.19, H; 10.81, Found (/%): C; 89.16, H; 10.76

### (2) Polymerization of polyadamantane compound and production of varnish for organic insulating film

A polymer was obtained in the same manner as Example 10(1), except that 5 g of the compound obtained in Example 10(1) was used instead of 5 g of a compound obtained by the same procedure as Example 4(1), used in Example 4(2). The weight-average molecular weight of the obtained polymer was 31,700. A 3 g portion of the obtained polymer was dissolved in 27 g of cyclopentanone and filtered with a filter to obtain a varnish for organic insulating film.

### (3) Production of interlayer insulating film and semiconductor device

The previously obtained varnish for organic insulating film was used for the same procedure as Example 1(3) to obtain an interlayer insulating film and semiconductor device.

### (Example 11)

### (1) [Synthesis of 3,3',5,5'-tetramethyl-7,7'-bis(3,5-diethynylphenyl)-1,1'-biadamantane]

After stirring 50 g (103.2 mmol) of 3,3',5,5'-tetramethyl-7,7'-dibromo-1,1'-biadamantane obtained by the same procedure as Example 7(1) and 1217 g (5161.6 mmol) of 1,3-dibromobenzene in a flask, 24.8 g (93.0 mmol) of aluminum(III) bromide was added dropwise in small portions at a time at 25°C under dry nitrogen. The mixture was raised to a temperature of 60°C and stirred for 8 hours, and then returned to room temperature to obtain a reaction mixture. The reaction mixture was added to 700 ml of a 5% hydrochloric acid aqueous solution and stirred. The aqueous layer was removed and the organic layer was added to 2000 ml of acetone. The precipitate was filtered and washed 3 times with 1000 ml of acetone to obtain 70 g of 3,3',5,5'-tetramethyl-7,7'-bis(3,5-dibromophenyl)-1,1'-biadamantane.

Next, 50 g (62.9 mmol) of the previously obtained 3,3',5,5'-tetramethyl-7,7'-bis(3,5-dibromophenyl)-1,1'-biadamantane, 3.53 g (5.0 mmol) of dichlorobistriphenylphosphinepalladium, 6.60 g (25.2 mmol) of triphenylphosphine, 4.79 g (25.2 mmol) of copper(II) iodide and 750 ml of triethylamine were added to a flask and stirred. The mixture was raised to 75°C, and then 37.1 g (377.7 mmol) of trimethylsilylacetylene was slowly added. After stirring at 75°C for 7 hours, the temperature was raised to 120°C to remove the triethylamine. After returning to room temperature, 1000 ml of dichloromethane was added to the reaction mixture and stirring was continued for 20 minutes. The precipitate was removed by filtration, and 1000 ml of a 5% hydrochloric acid aqueous solution was added to the filtrate for liquid separation. The organic layer was washed three times with 1000 ml of water, and then the solvent of the organic layer was removed under reduced pressure. The obtained compound was dissolved in 1500 ml of hexane. The insoluble portion was removed by filtration and the hexane in the filtrate was removed under reduced pressure. Next, 1000 ml of acetone was added and the precipitate was washed 3 times with acetone to obtain 43 g of 3,3',5,5'-tetramethyl-7,7'-bis(3,5-ditrimethylsilylethynylphenyl)-1,1'-biadamantane.

Next, 39.8 g (53.5 mmol) of the previously obtained 3,3',5,5'-tetramethyl-7,7'-bis(3,5-ditrimethylsilylethynylphenyl)-1,1'-biadamantane and 1.46 g (10.6 mmol) of potassium carbonate were stirred in a mixed solvent comprising 600 ml of tetrahydrofuran and 300 ml of methanol, for 4 hours under a nitrogen atmosphere at room temperature. This was added to 1000 ml of a 10% hydrochloric acid aqueous solution, and the precipitate was filtered and washed with 1000 ml of water and then with 1000 ml of acetone, after which it was dried to obtain 21.2 g of 3,3',5,5'-tetramethyl-7,7'-bis(3,5-diethynylphenyl)-1,1'-biadamantane.

The previously obtained compound was used for evaluation in the same manner as Example 1, whereby it was confirmed to be the same compound. The measurement results are shown below. Outer appearance: white solid
MS (FD)(m/z): 574 (M⁺)
Elemental analysis: Calculated (/%): C; 91.93, H; 8.07, Found (/%): C; 91.87, H; 8.00

### (2) Polymerization of polyadamantane compound and production of varnish for organic insulating film

A polymer was obtained in the same manner as Example 1(2), except that 5 g of the compound obtained in Example 11(1) was used instead of 5 g of a compound obtained in the same manner as Example 1(1), used in Example 1(2). The weight-average molecular weight of the obtained polymer was 103,200. A 3 g portion of the obtained polymer was dissolved in 27 g of cyclopentanone and filtered with a filter to obtain a varnish for organic insulating film.

### (3) Production of interlayer insulating film and semiconductor device

The previously obtained varnish for organic insulating film was used for the same procedure as Example 1(3) to obtain an interlayer insulating film and semiconductor device.

### (Example 12)

### (1) [Synthesis of 4,9-bis(3,5-diethynylphenyl)diamantane]

Except for using 35.7 g (103.2 mmol) of 4,9-dibromodiamantane instead of 50 g (103.2 mmol) of 3,3',5,5'-tetramethyl-7,7'-dibromo-1,1'-biadamantane, as in Example 11(1), the procedure was otherwise carried out in the same manner as Example 11(1) to obtain 38 g of 4,9-bis(3,5-diethynylphenyl)diamantane.

The previously obtained compound was used for evaluation in the same manner as Example 1, whereby it was confirmed to be the same compound. The measurement results are shown below. Outer appearance: white solid
MS (FD)(m/z): 436 (M⁺)
Elemental analysis: Calculated (/%): C; 93.54, H; 6.46, Found (/%): C; 93.61, H; 6.47

### (2) Polymerization of polyamantane compound and production of varnish for organic insulating film

A polymer was obtained in the same manner as Example 1(2), except that 5 g of the compound obtained in Example 12(1) was used instead of 5 g of a compound obtained by the same procedure as Example 1(1), used in Example 1(2). The weight-average molecular weight of the obtained polymer was 120,800. A 3 g portion of the obtained polymer was dissolved in 27 g of cyclopentanone and filtered with a filter to obtain a varnish for organic insulating film.

### (3) Production of interlayer insulating film and semiconductor device

The previously obtained varnish for organic insulating film was used for the same procedure as Example 1(3) to obtain an interlayer insulating film and semiconductor device.

### (Example 13)

### (1) [Synthesis of 9,9'-bis(3,5-diethynylphenyl)-4,4'-bi(diamantane)]

Except for using 80.2 g (0.3 mol) of 4-bromo-diamantane instead of 64.5 g (0.3 mol) of 1-bromoadamantane and using 35 g (0.22 mol) of bromine, as in Example 6(1), the procedure was otherwise carried out in the same manner as Example 6(1) to obtain 70 g of a product. Bromo group absorption was seen at 690-515 cm⁻¹ by IR analysis and the molecular weight by mass spectrometry was 532, thus indicating that the product was 9,9'-dibromo-4,4'-bi(diamantane).

Except for using 54.9 g (103.2 mmol) of the previously obtained 9,9'-dibromo-4,4'-bi(diamantane) instead of the 50 g (103.2 mmol) of 3,3',5,5'-tetramethyl-7,7'-dibromo-1,1'-biadamantane used in Example 11(1), the procedure was otherwise carried out as in Example 11(1) to obtain 31 g of 9,9'-bis(3,5-diethynylphenyl)-4,4'-bi(diamantane).

The previously obtained compound was used for evaluation in the same manner as Example 1, whereby it was confirmed to be the same compound. The measurement results are shown below. Outer appearance: white solid
MS (FD)(m/z): 622 (M⁺)
Elemental analysis: Calculated (/%): C; 92.56, H; 7.44, Found (/%): C; 92.12, H; 7.30

### (2) Polymerization of poly(polyamantane) compound and production of varnish for organic insulating film

A polymer was obtained in the same manner as Example 1(2), except that 5 g of the compound obtained in Example 13(1) was used instead of 5 g of a compound obtained by the same procedure as Example 1(1), used in Example 1(2). The weight-average molecular weight of the obtained polymer was 158,900. A 3 g portion of the obtained polymer was dissolved in 27 g of cyclopentanone and filtered with a filter to obtain a varnish for organic insulating film.

### (3) Production of interlayer insulating film and semiconductor device

The previously obtained varnish for organic insulating film was used for the same procedure as Example 1(3) to obtain an interlayer insulating film and semiconductor device.

### (Example 14)

### (1) [Synthesis of 3,3',5,5'-tetramethyl-7,7'-bis(3,5-diphenylethynylphenyl)-1,1'-biadamantane]

Except for using 10.6 g (13.4 mmol) of the 3,3',5,5'-tetramethyl-7,7'-bis(3,5-dibromophenyl)-1,1'-biadamantane obtained during the synthesis of Example 11(1) instead of the 10 g (26.7 mmol) of 3,3'-diethynyl-5,5',7,7'-tetramethyl-1,1'-biadamantane in Example 9(1), and using 8.1 g (79.6 mmol) of ethynylbenzene instead of 12.5 g (79.6 mmol) of bromobenzene, the procedure was otherwise carried out in the same manner to obtain 11.6 g of 3,3',5,5'-tetramethyl-7,7'-bis(3,5-diphenylethynylphenyl)-1,1'-biadamantane.

The previously obtained compound was used for evaluation in the same manner as Example 1, whereby it was confirmed to be the same compound. The measurement results are shown below. Outer appearance: white solid
MS (FD)(m/z): 878 (M⁺)
Elemental analysis: Calculated (/%): C; 92.89, H; 7.11, Found (/%): C; 92.95, H; 7.05

### (2) Polymerization of polyadamantane compound and production of varnish for organic insulating film

A polymer was obtained in the same manner as Example 5(2), except that 5 g of the compound obtained in Example 14(1) was used instead of 5 g of a compound obtained by the same procedure as Example 5(1), used in Example 5(2). The weight-average molecular weight of the obtained polymer was 18,600. A 3 g portion of the obtained polymer was dissolved in 27 g of cyclopentanone and filtered with a filter to obtain a varnish for organic insulating film.

### (3) Production of interlayer insulating film and semiconductor device

The previously obtained varnish for organic insulating film was used for the same procedure as Example 1(3) to obtain an interlayer insulating film and semiconductor device.

### (Example 15)

### (1) Polymerization of polyadamantane compound and production of varnish for organic insulating film

A 3 g portion of the 3,3',5,5'-tetraethynyl-1,1'-biadamantane obtained in Example 6(1) was dissolved in 35 ml of pyridine and 35 ml of methanol in a flask, and then 7 g of copper(II) acetate was added and reaction was conducted at 80°C for 1 hour under a nitrogen atmosphere. The reaction mixture was added dropwise to 500 ml of a 2 mol/l hydrochloric acid aqueous solution, and the precipitate was recovered to obtain a polymer. The weight-average molecular weight of the obtained polymer was 109,500. A 2 g portion of the obtained polymer was dissolved in 18 g of cyclopentanone and filtered with a filter to obtain a varnish for organic insulating film.

### (2) Production of interlayer insulating film and semiconductor device

The previously obtained varnish for organic insulating film was used for the same procedure as Example 1(3) to obtain an interlayer insulating film and semiconductor device.

### (Example 16)

### (1) Production of varnish for organic insulating film comprising polyadamantane compound and polymer

After dissolving 1.5 g of the 3,3',5,5'-tetraethynyl-1,1'-biadamantane polymer obtained in Example 6(1) and 1.5 g of the 3,3'-bis(phenylethynyl)-5,5',7,7'-tetramethyl-1,1'-biadamantane obtained in Example 9(1) in 27 g of cyclopentanone, the solution was filtered with a filter to produce a varnish for organic insulating film.

### (2) Production of interlayer insulating film and semiconductor device

The previously obtained varnish for organic insulating film was used for the same procedure as Example 1(3) to obtain an interlayer insulating film and semiconductor device.

### (Example 17)

### (1) [Synthesis of 3,3',5,5'-tetramethyl-7,7'-bis(2-propenyloxy)-1,1'-biadamantane]

In a 1 L volumetric flask there were loaded 43.4 g (100 mmol) of 3,3',5,5'-tetramethyl-7,7'-bis(2-propynyloxy)-1,1'-biadamantane, 193.7 g (1500 mmol) of quinoline, 3.19 g (1.5 mmol) of 5% palladium calcium carbonate and 300 ml of tetrahydrofuran, and the mixture was stirred under hydrogen while being kept at 27°C. Upon consumption of 4.9 L (200 mmol) of hydrogen, nitrogen was introduced to suspend the reaction. After filtering the reaction mixture, the solvent was removed under reduced pressure and the obtained solid was washed with a mixture of acetone and water (volume ratio = 2:1) and dried under reduced pressure at 60°C to obtain 39.2 g of 3,3',5,5'-tetramethyl-7,7'-bis(2-propenyloxy)-1,1'-biadamantane.

The previously obtained compound was used for evaluation in the same manner as Example 1, whereby it was confirmed to be the same compound. The measurement results are shown below. Outer appearance: white solid
MS (FD)(m/z): 438 (M⁺)
Elemental analysis: Calculated (/%): C; 82.14, H; 10.57, O; 7.29, Found (/%): C; 82.20, H; 10.64, O; 7.16

### (2) Polymerization of polyadamantane compound and production of varnish for organic insulating film

A polymer was obtained in the same manner as Example 4(2), except that 5 g of the compound obtained in Example 14(1) was used instead of 5 g of a compound obtained by the same procedure as Example 4(1), used in Example 4(2). The weight-average molecular weight of the obtained polymer was 23,500. A 3 g portion of the obtained polymer was dissolved in 27 g of cyclopentanone and filtered with a filter to obtain a varnish for organic insulating film.

### (3) Production of interlayer insulating film and semiconductor device

The previously obtained varnish for organic insulating film was used for the same procedure as Example 1(3) to obtain an interlayer insulating film and semiconductor device.

### (Example 18)

### (1) [Synthesis of 3,3',5,5'-tetravinyl-1,1'-biadamantane]

Except for using 18.3 g (50 mmol) of 3,3',5,5'-tetraethynyl-1,1'-biadamantane obtained by the same procedure as Example 6(1) instead of the 43.4 g (100 mmol) of 3,3',5,5'-tetramethyl-7,7'-bis(2-propynyloxy)-1,1'-biadamantane as in Example 17(1), the procedure was otherwise carried out as in Example 17(1) to obtain 17.1 g of 3,3',5,5'-tetravinyl-1,1'-biadamantane.

The previously obtained compound was used for evaluation in the same manner as Example 1, whereby it was confirmed to be the same compound. The measurement results are shown below. Outer appearance: white solid
MS (FD)(m/z): 374 (M⁺)
Elemental analysis: Calculated (/%): C; 89.78, H; 10.22, Found (/%): C; 89.72, H; 10.20

### (2) Polymerization of polyadamantane compound and production of varnish for organic insulating film

After dissolving 3 g of the compound obtained in Example 18(1) in 70 ml of tetrahydrofuran which had the moisture sufficiently removed by distillation, 2 ml of a 1 mol/L trifluoromethanesulfonic acid/hexane solution was added, reaction was conducted at 0°C for 3 hours under dry nitrogen, the reaction mixture was added dropwise into a 10-fold volume of methanol, and the precipitate was collected and dried to obtain a polymer. The weight-average molecular weight of the obtained polymer was 82,900. A 2 g portion of the obtained polymer was dissolved in 18 g of cyclohexanone and filtered with a filter to obtain a varnish for organic insulating film.

### (3) Production of interlayer insulating film and semiconductor device

The previously obtained varnish for organic insulating film was used for the same procedure as Example 1(3) to obtain an interlayer insulating film and semiconductor device.

### (Example 19)

### (1) [Synthesis of 3,3'-divinyl-5,5',7,7'-tetramethyl-1,1'-biadamantane]

Except for using 37.5 g (100 mmol) of 3,3'-diethynyl-5,5',7,7'-tetramethyl-1,1'-biadamantane obtained by the same procedure as Example 7(1) instead of the 43.4 g (100 mmol) of 3,3',5,5'-tetramethyl-7,7'-bis(2-propynyloxy)-1,1'-biadamantane as in Example 17(1), the procedure was otherwise carried out as in Example 17(1) to obtain 32.8 g of 3,3'-divinyl-5,5',7,7'-tetramethyl-1,1'-biadamantane.

The previously obtained compound was used for evaluation in the same manner as Example 1, whereby it was confirmed to be the same compound. The measurement results are shown below. Outer appearance: white solid
MS (FD)(m/z): 378 (M⁺)
Elemental analysis: Calculated (/%): C; 88.82, H; 11.18, Found (/%): C; 88.76, H; 11.15

### (2) Polymerization of polyadamantane compound and production of varnish for organic insulating film

After dissolving 3 g of the compound obtained in Example 19(1) in 70 ml of tetrahydrofuran which had the moisture sufficiently removed by distillation, 1 ml of a 1 mol/L n-butyllithium/hexane solution was added, reaction was conducted at 0°C for 3 hours under dry nitrogen, the reaction mixture was added dropwise into a 10-fold volume of methanol, and the precipitate was collected and dried to obtain a polymer. The weight-average molecular weight of the obtained polymer was 29,900. A 2 g portion of the obtained polymer was dissolved in 18 g of cyclohexanone and filtered with a filter to obtain a varnish for organic insulating film.

### (3) Production of interlayer insulating film and semiconductor device

The previously obtained varnish for organic insulating film was used for the same procedure as Example 1(3) to obtain an interlayer insulating film and semiconductor device.

### (Example 20)

### (1) [Synthesis of 3,3',5,5'-tetramethyl-7,7'-bis(3,5-divinylphenyl)-1,1'-biadamantane]

Except for using 28.7 g (50 mmol) of 3,3',5,5'-tetramethyl-7,7'-bis(3,5-diethynylphenyl)-1,1'-biadamantane obtained by the same procedure as Example 11(1) instead of the 43.4 g (100 mmol) of 3,3',5,5'-tetramethyl-7,7'-bis(2-propynyloxy)-1,1'-biadamantane as in Example 17(1), the procedure was otherwise carried out as in Example 17(1) to obtain 25.1 g of 3,3',5,5'-tetramethyl-7,7'-bis(3,5-divinylphenyl)-1,1'-biadamantane.

The previously obtained compound was used for evaluation in the same manner as Example 1, whereby it was confirmed to be the same compound. The measurement results are shown below. Outer appearance: white solid
MS (FD)(m/z): 582 (M⁺)
Elemental analysis: Calculated (/%): C; 90.66, H; 9.34, Found (/%): C; 90.60, H; 9.39

### (2) Polymerization of polyadamantane compound and production of varnish for organic insulating film

A polymer was obtained in the same manner as Example 1(2), except that 5 g of the compound obtained in Example 20(1) was used instead of 5 g of a compound obtained by the same procedure as Example 1(1), used in Example 1(2). The weight-average molecular weight of the obtained polymer was 129,200. A 3 g portion of the obtained polymer was dissolved in 27 g of cyclopentanone and filtered with a filter to obtain a varnish for organic insulating film.

### (3) Production of interlayer insulating film and semiconductor device

The previously obtained varnish for organic insulating film was used for the same procedure as Example 1(3) to obtain an interlayer insulating film and semiconductor device.

### (Comparative Example 1)

In a flask there were added 60 ml of tetrahydrofuran, 1.73 g of tetrakistriphenylphosphinepalladium, 0.72 g of copper(II) iodide, 10 ml of piperidine and 54.7 g (120 mmol) of 1,2,4-triiodobenzene. Next, 32.74 g (150 mmol) of 4,4'-diethynyldiphenyl ether was added and reaction was conducted at 25°C for 20 hours. The reaction mixture was dropped into 1 L of acetic acid and the precipitate was collected and dried to obtain a polymer. The weight-average molecular weight of the obtained polymer was 102,900. A 3 g portion of the obtained polymer was dissolved in 27 g of anisole and filtered with a filter to obtain a varnish for organic insulating film. The varnish for organic insulating film was used for the same procedure as Example 1(3) to form an interlayer insulating film and obtain a semiconductor device.

The evaluation described below was carried out for the interlayer insulating films obtained in Examples 1-20 and Comparative Example 1. The evaluated parameters are shown together with the methods used. The results are shown in Table 1.

### 1. Elastic modulus

Each obtained insulating film was measured using an Ultramicro Hardness Meter ENT-1100 by Elionix Co., Ltd., with a maximum load of 10 mg and a load speed of 1 mg/sec.

### 2. Relative permittivity

Following the procedure of JIS-K6911, a HP-4284A Precision LCR Meter by Hewlett Packard was used for volume measurement of the obtained insulating film at a frequency of 100 kHz, and the relative permittivity was calculated by the following formula. Relative permittivity = (Measured volume value x film thickness)/(vacuum permittivity x measured area)

### 3. Heat resistance

The heat resistance was evaluated by the glass transition temperature and thermal decomposition temperature. For the glass transition temperature, the obtained insulating film was measured with a dynamic viscoelasticity measuring apparatus (DMS6100 by Seiko Instruments, Inc.), under conditions with a 300 mL/min. nitrogen gas flow, a temperature-elevating rate of 3°C/min. and a frequency of 1 Hz, and the tanδ peak top temperature was recorded as the glass transition temperature.

For the thermal decomposition temperature, the obtained insulating film was measured with a TG/DTA measuring apparatus (TG/DTA220 by Seiko Instruments, Inc.) under conditions with a 200 mL/min nitrogen gas flow and a temperature-elevating rate of 10°C/min, and the temperature at which the mass reduction reached 5% was recorded as the thermal decomposition temperature.

**Table 1**

| | Elastic modulus (GPa) | Relative permittivity | Thermal decomposition temperature (°C) | Glass transition temperature |
|---|---|---|---|---|
| Example 1 | 8.72 | 2.34 | 463 | ≥400 |
| Example 2 | 8.87 | 2.37 | 478 | ≥400 |
| Example 3 | 7.21 | 2.50 | 489 | ≥400 |
| Example 4 | 7.70 | 2.68 | 496 | ≥400 |
| Example 5 | 7.09 | 2.72 | 528 | ≥400 |
| Example 6 | 9.57 | 2.30 | 532 | ≥400 |
| Example 7 | 8.94 | 2.24 | 461 | ≥400 |
| Example 8 | 7.00 | 2.38 | 451 | ≥400 |
| Example 9 | 7.03 | 2.52 | 490 | ≥400 |
| Example 10 | 8.12 | 2.16 | 482 | ≥400 |
| Example 11 | 8.50 | 2.48 | 510 | ≥400 |
| Example 12 | 7.53 | 2.46 | 487 | ≥400 |
| Example 13 | 7.22 | 2.27 | 466 | ≥400 |
| Example 14 | 8.21 | 2.63 | 537 | ≥400 |
| Example 15 | 7.65 | 2.25 | 458 | ≥400 |
| Example 16 | 8.55 | 2.42 | 501 | ≥400 |
| Example 17 | 7.01 | 2.30 | 448 | ≥400 |
| Example 18 | 8.41 | 2.29 | 462 | ≥400 |
| Example 19 | 7.92 | 2.21 | 459 | ≥400 |
| Example 20 | 7.58 | 2.48 | 480 | ≥400 |
| Comp. Ex. 1 | 6.74 | 2.93 | 450 | ≥400 |

According to Table 1, Examples 1-20 had higher elastic moduli and superior mechanical properties compared to the comparative example. In addition, Examples 1-20 had lower relative permittivity values and more excellent dielectric characteristics than the comparative example. Examples 1-20 also had higher thermal decomposition temperatures and more excellent heat resistances than the comparative example.

The wiring delay speeds of the obtained semiconductor devices were evaluated.

The wiring delays of semiconductor devices obtained using the interlayer insulating films of Examples 1-20 and a semiconductor device with an SiO₂ insulating film having the same construction as these semiconductor devices were compared. The signal delay time calculated from the transmission frequency of a ring oscillator was used as the standard for evaluation. Upon comparing both, the semiconductor devices obtained according to the invention were confirmed to have lower wiring delays than the semiconductor device with the SiO₂ insulating film, with an average speed increase of about 25%.

### Industrial Applicability

According to the invention it is possible to provide organic insulating materials exhibiting low permittivity, high heat resistance and high mechanical strength. The organic insulating materials, and organic insulating films obtained using varnishes for organic insulating film containing them, exhibit excellent heat-resistant properties, mechanical properties and electrical characteristics, and especially low permittivity, and therefore semiconductor devices employing them can reduce wiring delay.

## Claims

1. An organic insulating material comprising a compound represented by general formula (1), or a polymer obtained by polymerizing a compound represented by general formula (1), or a mixture of a compound represented by general formula (1) and said polymer. 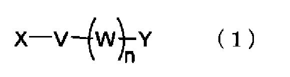 (In formula (1), X and Y each independently represent one or more groups with polymerizable functional groups. V and W each represent a group having an adamantane or polyamantane structure, and they may be the same or different. The letter n represents an integer of 0 or greater.)

2. An organic insulating material according to claim 1, wherein the compound represented by general formula (1) is a compound represented by general formula (2). 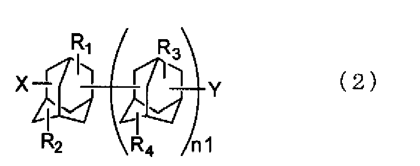 (In formula (2), X and Y each independently represent one or more groups with polymerizable functional groups. R₁-R₄ each independently represent hydrogen or an organic group, and they may be the same or different from each other. The symbol n1 represents an integer of 0 or greater. When n1 is an integer of 2 or greater, R₃ and R₄ may be the same or different in each adamantane structure.)

3. An organic insulating material according to claim 2, wherein the compound represented by general formula (2) is a biadamantane compound.

4. An organic insulating material according to claim 3, wherein the biadamantane compound has a 1,1'-biadamantane skeleton.

5. An organic insulating material according to any one of claims 1 to 4, wherein the compound represented by general formula (1) has a group with a polymerizable unsaturated bond group as X and/or Y.

6. An organic insulating material according to claim 5, wherein the polymerizable unsaturated bond group is an acetylene bond group or vinyl bond group.

7. An organic insulating material according to claim 6, wherein X and Y in the compound represented by general formula (1) each independently have one or more groups selected from among groups represented by general formulas (3)-(8).
-Z(̵O-R₅-C≡C-R₆)ₘ₁ (3)
(In formula (3), Z represents a single bond or an aromatic group, R₅ represents an aliphatic group, and R₆ represents hydrogen or an organic group. When Z is a single bond m1 is 1, and when Z is an aromatic group m1 is 1 or 2.)
-C=C-R₆ (4)
(In formula (4), R₆ represents hydrogen or an organic group.) 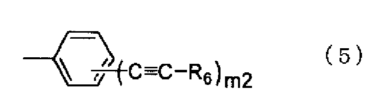 (In formula (5), R₆ represents hydrogen or an organic group. The symbol m2 represents an integer of 1-5.) 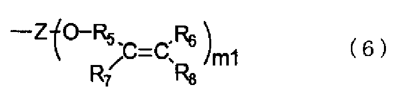 (In formula (6), Z represents a single bond or an aromatic group, R₅ represents an aliphatic group and R₆-R₈ represent hydrogen or organic groups and each independently may be the same or different. When Z is a single bond m1 is 1, and when Z is an aromatic group m1 is 1 or 2.) 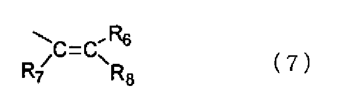 In formula (7), R₆-R₈ represent hydrogen or organic groups and each independently may be the same or different.) 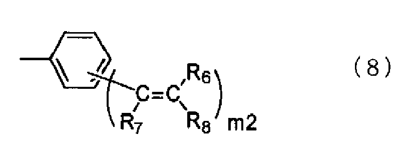 In formula (8), R₆-R₈ represent hydrogen or organic groups and each independently may be the same or different. The symbol m2 represents an integer of 1-5.)

8. A varnish for organic insulating film comprising an organic insulating material according to any one of claims 1 to 7 and an organic solvent.

9. An organic insulating film obtained by using an organic insulating material according to any one of claims 1 to 7 or a varnish for organic insulating film according to claim 8, for crosslinking reaction by heating, active energy beam irradiation, or heating and active energy beam irradiation.

10. A semiconductor device provided with an organic insulating film according to claim 9.
